# EUROPEAN PATENT APPLICATION

(11) **EP 4 840 476 A1**
(43) Date of publication of application: **18.11.2026**
(21) Application number: 24916532.5
(22) Date of filing: 29.10.2024
(51) Int. Cl.: A61L 24/10, A61L 24/00

(54) **SILK FIBROIN COMPLEX LOADED WITH POLYHYDROXY PHENOLIC COMPOUND, PREPARATION METHOD THEREFOR, AND USE THEREOF**

(30) Priority: 11.01.2024 CN 202410041610
(71) Applicant: Nanjing University Of Chinese Medicine, Nanjing, Jiangsu 210023 (CN)
(72) Inventor: CHENG, Jianming, Nanjing, Jiangsu 210023 (CN); GU, Wei, Nanjing, Jiangsu 210023 (CN); JI, Jing, Nanjing, Jiangsu 210023 (CN); ZHANG, Yunyu, Nanjing, Jiangsu 210023 (CN); QI, Shuyang, Nanjing, Jiangsu 210023 (CN); TANG, Ruoyan, Nanjing, Jiangsu 210023 (CN); SONG, Bohui, Nanjing, Jiangsu 210023 (CN); XU, Jiarong, Nanjing, Jiangsu 210023 (CN)
(74) Representative: Bayramoglu et al.
(86) International application number: PCT/CN2024/127986
(87) International publication number: WO 2025/148481

(57) **Abstract**

Aiming at the inadequacy of existing hemostatic materials for large-scale trauma and serious bleeding, the present invention provides a silk fibroin complex loaded with a polyhydroxy phenolic compound and a preparation method therefor. The complex is prepared by self-assembly of the polyhydroxy phenolic compound and the silk fibroin. The silk fibroin is subjected to a β-sheet conformation transformation process. The polyhydroxy phenolic compound is preferably an insoluble compound, most preferably ellagic acid. The feeding ratio of the polyhydroxy phenolic compound to the silk fibroin is a mass ratio of <1:1, preferably 1:2 to 1:16, more preferably 1:4. The hemostatic ability of the complex is significantly superior to that of the polyhydroxy phenolic compound, the silk fibroin, Yunnan Baiyao, tranexamic acid, chitosan hemostatic powder, and the like. Meanwhile, the complex has a larger particle size and mass than the silk fibroin, and can rapidly settle to a bleeding site in blood. The complex exhibits no cytotoxicity, is blood-insoluble, and is capable of self-degradation in vivo, demonstrating high safety. Meanwhile, the complex has the characteristics of wound healing, antibiosis, infection resistance, adhesion resistance, and the like, and can be applied to the preparation of corresponding formulations.

## Description

### TECHNICAL FIELD

The present invention pertains to the technical field of materials, and particularly relates to a silk fibroin complex loaded with a polyhydroxy phenolic compound, a preparation method therefor, and a use thereof.

### BACKGROUND TECHNOLOGY

Hemorrhage refers to sudden and severe blood leakage caused by vascular rupture. It constitutes one of the most common causes of death worldwide. Deaths from surgical hemorrhage and combat-induced hemorrhage are the most significant cases. According to statistics, severe hemorrhage accounts for over 35% of pre-hospital deaths and over 40% of deaths within 24 hours after injury. The excessive blood loss caused by modern warfare has significantly increased. Deaths within 30 minutes after a combat injury account for 80% of all combat-related deaths. On the battlefield, the first hour after a wounded person is injured is known as the "golden hour" for rescue efforts. Therefore, the cost and technical research of hemostatic materials not only impose a heavy burden on the global economy, but also constitute an urgent challenge to be addressed in all emergency rescue scenarios.

At present, the hemostasis market is dominated by absorbable hemostatic materials, including gauzes, sponges, sprays, hemostatic powders, and the like. Their main components are mostly natural polymers such as cellulose, chitosan and fibrin. The major advantages of these materials include short hemostasis time, biodegradability, relatively mild foreign body reaction, no need for secondary surgery for removal, and avoidance of secondary trauma to patients. Nevertheless, several drawbacks restrict their clinical application, including high cost, inconsistent absorption time, limited adaptability, and inadequacy for large-scale trauma and serious bleeding. For example, oxidized regenerated cellulose (ORC)-based hemostatic materials contain abundant acidic groups and reach a pH value of 1.7 when fully saturated with water. Under acidic conditions, despite their moderate antibacterial activity, residual fragments of such materials left in wounds may trigger severe inflammatory responses, inhibit bone tissue regeneration, and even lead to the formation of granulomas, abscesses and other lesions. Therefore, it is necessary to develop an absorbable hemostatic material that is rapid, efficient, cost-effective, and free of toxic and side effects.

Silk fibroin, a natural macromolecular protein extracted from silkworm silk, has excellent in vivo degradability, biocompatibility and anti-inflammatory properties. In terms of hemostatic mechanisms, the silk fibroin activates coagulation factor XII, thereby triggering intrinsic coagulation cascade reactions. It is a biomacromolecule consisting of 5,509 amino acids, composed of a heavy chain (391kDa) and a light chain (26kDa), which are linked together via disulfide bonds. When the silk fibroin dissolved in a lithium bromide (LiBr) solution or a ternary solution is poured into an excess polar organic solvent such as alcohol, a white protein complex suspension will be formed immediately. When the silk fibroin remains in the solution, the peptide chains adopt a random coil conformation (i.e., α-helix) due to multiple interactions between the side chains of the silk fibroin, salt ions and water molecules. This state also represents the native conformation of silk fibroin. In this state, the silk fibroin lacks the ability to self-assemble with a polyhydroxy phenolic compound. Owing to the large steric hindrance of benzene rings on amino acid residues, the polyhydroxy phenolic compound can hardly integrate into a α-helix structure and thus remain exposed on the exterior of the silk fibroin molecule. When an antisolvent such as ethanol is applied, the peptide chain will be induced to rapidly fold into more stable β-sheet assemblies, and the β-sheet conformation will expose the hydrophobic amino acid residues, which can enhance the encapsulation of hydrophobic drugs via hydrophobic interactions and π-π stacking, facilitating subsequent self-assembly with the polyhydroxy phenolic compound.

The silk fibroin hemostatic agents available in the market at present fail to overcome the barrier posed by the hydration layer at the blood interface, exhibit poor adhesion to bleeding wounds, and cannot meet the requirements for strong tissue adhesion under wet conditions. In contrast, the polyhydroxy phenolic compound can form a secondary cross-linking structure with the silk fibroin, thereby recruiting more platelets and reducing the risk of the silk fibroin being washed away by blood. Meanwhile, the polyhydroxy phenolic compound and the silk fibroin exert a synergistic effect to promote endogenous hemostasis.

### CONTENT OF THE INVENTION

The objective of the present invention is to address the drawbacks of the prior art, including inadequacy for large-scale trauma and serious bleeding, insufficient blood absorption and seepage control capacities, and inability to treat irregular wounds, and provide a silk fibroin complex loaded with a polyhydroxy phenolic compound, a preparation method therefor, and a use thereof. The technical solution is as follows:
The present invention provides a silk fibroin complex loaded with a polyhydroxy phenolic compound. The complex is prepared by self-assembly of the polyhydroxy phenolic compound and the silk fibroin. The silk fibroin is subjected to a β-sheet conformation transformation process. The feeding ratio of the polyhydroxy phenolic compound to the silk fibroin is a mass ratio of <1:1, preferably 1:2 to 1:16, most preferably 1:4.

As a further improvement, the polyhydroxy phenolic compound is one or more of resveratrol (RES), quercetin (QUE), baicalein (BAE), kaempferol (KAE), lignan, curcumin (CUR), ellagic acid (EA), chlorogenic acid (CGA), ferulic acid (FA), caffeic acid (CFA), syringic acid (SA), sinapic acid, rutin (RUT), myricetin (MYR), magnolol acid (MA), fisetin (FIS), puerarin (PUE), rhein (RHE), lonicerin (Lon), proanthocyanidins (PC), p-coumaric acid (p-CA), vanillic acid (VA), hesperetin (Hes), naringenin (NAR), luteolin (Lut), genistein (Gen), emodin (Emo), oleanolic acid (Ola), epigallocatechin gallate (EGCG), acaciin, tea polyphenols (TP), epigallocatechin (EGC), danshensu (DS), gallic acid (GA), pyrogallic acid (PA), catechin (C) and tannic acid (TA).

As a still further improvement, the polyhydroxy phenolic compound is an insoluble polyhydroxy phenolic compound, preferably one or more of resveratrol, quercetin, baicalein, kaempferol, lignan, curcumin, ellagic acid, chlorogenic acid, ferulic acid, caffeic acid, syringic acid, sinapic acid, rutin, myricetin, magnolol acid, fisetin, puerarin, rhein, lonicerin, proanthocyanidins, p-coumaric acid, vanillic acid, hesperetin, naringenin, luteolin, genistein, emodin, oleanolic acid and epigallocatechin gallate, most preferably ellagic acid.

The feed ratio of ellagic acid as the most preferred to the silk fibroin is a mass ratio of preferably 1:2 to 1:16, more preferably 1:4.

The present invention further provides a preparation method for the foregoing silk fibroin complex, comprising a step of silk fibroin conformation transformation and a step of self-assembly of the silk fibroin and the polyhydroxy phenolic compound: step (1): the silk fibroin is subjected to a conformation transformation process from α-helix to β-sheet to obtain a silk fibroin solution, wherein the conformation transformation can be achieved by either chemical or physical methods, the chemical methods include promoting the transformation through polyols, polylactic acid, metal ions, pH or hydroxypropyl methylcellulose, and the physical methods include promoting the transformation through high temperature, hydration and pressure, ultra-low temperature storage, lyophilizing, shear force, ultrasound, eddy current, laser irradiation, and high-pressure carbon dioxide treatment; step (2): after being dissolved or suspended, the polyhydroxy phenolic compound is mixed with the silk fibroin solution obtained in step (1) and stirred to induce self-assembly, thereby obtaining a silk fibroin complex loaded with the polyhydroxy phenolic compound. In step (1), preferably, a polyol is used to promote the transformation of the β-sheet conformation of the silk fibroin. The more preferred polyol is ethanol.

As an improvement to the preparation method, the polyhydroxy phenolic compound is an insoluble polyhydroxy phenolic compound, the insoluble polyhydroxy phenolic compound in step (2) is dissolved or suspended in an organic solvent, and the organic solvent is selected from methanol, ethanol, propanol, propylene glycol, glycerol, n-butanol and isobutanol.

When the insoluble polyhydroxy phenolic compound is ellagic acid, the preferred organic solvent is propylene glycol. After the ellagic acid suspension is incubated with the silk fibroin in step (2), the final concentration of propylene glycol in the resulting solution is 5% to 20%, and the most preferred concentration is 10%.

The present invention provides a use of the foregoing silk fibroin complex in the preparation of a formulation with a hemostatic efficacy, wherein the formulation comprises the foregoing silk fibroin complex and pharmaceutically acceptable excipients. The dosage forms of the formulation include gauze, sponge, spun fiber, spray, powder, granule, gel, sealant, ointment, film, patch and suppository.

The present invention also provides a use of the foregoing silk fibroin complex in the preparation of a formulation with the efficacies of wound healing, and/or infection resistance, and/or adhesion resistance, wherein the formulation comprises the foregoing silk fibroin complex and pharmaceutically acceptable excipients.

### Advantages of the present invention:

The silk fibroin complex loaded with a polyhydroxy phenolic compound developed by the present invention serves as an effective hemostatic agent. Its hemostatic and coagulation ability is significantly superior to that of the polyhydroxy phenolic compound and the silk fibroin alone, indicating that these two materials have a synergistic effect. In addition, the hemostatic effect of this hemostatic agent is significantly superior to that of many commercially available hemostatic products, such as Yunnan Baiyao, tranexamic acid, and chitosan hemostatic powder. The silk fibroin complex provided by the present invention has a larger particle size and mass, greater mechanical strength and stronger wet-state tissue adhesion compared to the silk fibroin and has a higher blood settlement rate and can rapidly settle to a bleeding site compared to other drugs, while the powders of Yunnan Baiyao, tranexamic acid, chitosan hemostatic powder, and silk fibroin are lighter and more likely to float on the surface of the whole blood and be washed away by the blood flow, thereby affecting their hemostatic effect. The silk fibroin complex exhibits no cytotoxicity, is blood-insoluble, and is capable of self-degradation in vivo, demonstrating high safety. Meanwhile, the silk fibroin complex has the efficacies of wound healing, antibiosis, infection resistance, adhesion resistance, and the like.

### DESCRIPTION OF THE DRAWINGS

The present invention will be further described below by referring to the accompanying drawings:
FIG. 1 Fourier Transform Infrared Spectroscopy (FTIR).
FIG. 2-FIG. 4 Blood Viscosity Comparison Charts (n=3), of which FIG. A: *, compared with the blank control group, **P*<0.05, ***P*<0.01, ****P*<0.001; #, compared with the β-SF-EA group, #*P*<0.05, ##*P*<0.01, ###*P*<0.001. Compared with the β-SF-polyhydroxy phenolic compound group, **P*<0.05, ***P*<0.01, ****P*<0.001, e.g.: compared with the β-SF-EA group, **P*<0.05, ***P*<0.01, ****P*<0.001.
FIG. 5 Blood Coagulation Time Comparison Chart (n=3), *, compared with the β-SF-EA group, **P*<0.05, ***P*<0.01, ****P*<0.001.
FIG. 6 Drug Loading Comparison Chart (n=3). *, compared with the β-SF-EA group, **P*<0.05, ***P*<0.01, ****P*<0.001; ND means Not Detected.
FIG. 7-FIG. 8 Drug Loading Comparison Charts (n=3). *, compared with the water group, **P*<0.05, ***P*<0.01, ****P*<0.001.
FIG. 9 Fourier Transform Infrared Spectroscopy (FTIR), where EA and β-SF are physically mixed at a mass ratio of 1:1 (w/w).
FIG. 10 Molecular Docking Diagram. L74 stands for leucine (Leu) at position 74, and D27 stands for aspartic acid (Asp) at position 27.
FIG. 11 Representation: (A) FTIR spectrum, (B) XRD, (C) TG, (D) 1H NMR.
FIG. 12 Blood Adsorption Experiment, where a represents blank control, b represents β-SF, c represents EA, d represents Yunnan Baiyao, e represents tranexamic acid, and f represents β-SF-EA, the same in FIG. 13.
FIG. 13 Tube Tilting Coagulation Experiment Diagram.
FIG. 14 Blood Coagulation Index (BCI) Chart (n=3).
FIG. 15 Rat Tail Amputation Experiment, where FIG. A shows the bleeding images of rats applied with drug powder immediately after tail amputation, and Figure B shows the hemostasis time charts after tail amputation; *, compared with the β-SF-EA group, **P*<0.05, ***P*<0.01, ****P*<0.001.
FIG. 16 Charts for Bleeding Time of Liver and Femoral Artery in Rats (n=3). *, compared with the β-SF-EA group, **P*<0.05, ***P*<0.01, ****P*<0.001.
FIG. 17 Charts for Relative Growth Rates of LO2 Cells and L929 Cells After 48 Hours of Co-Incubation with a Drug (n=6).
FIG. 18 Chart for in Vitro Degradation of β-SF-EA (n=3).
FIG. 19 Representation of Hemostatic Powder Containing Ellagic Acid-Loaded Silk Fibroin Nanoparticles (β-SFN-EA).
FIG. 20 Charts for Bleeding Time of the Ear Marginal Artery, Liver and Femoral Artery in Rabbits (n=6).
FIG. 21 Charts for Bleeding Volume of the Ear Marginal Artery, Liver and Femoral Artery in Rabbits (n=6).
FIG. 22 In Vivo Biocompatibility: H&E and Masson Trichrome Staining Images of the Surrounding Tissues Separated 7, 15, and 30 Days After Subcutaneous Implantation of the Material.
FIG. 23 Comparison Chart of Wound Closure Rates (n=3), *, compared with the blank control group, **P*<0.05, ***P*<0.01, ****P*<0.001; #, compared with the β-SF-EA group, #*P*<0.05, ##*P*<0.01, ###*P*<0.001.
FIG. 24 H&E Staining of Wounds.
FIG. 25 H&E Staining of Femoral Artery Wounds in Rabbits.
FIG. 26 Comparison Chart of Liver Healing Rates (n=3), *, compared with the β-SF-EA group, **P*<0.05, ***P*<0.01, ****P*<0.001.

### SPECIFIC IMPLEMENTATIONS

### Embodiment 1: Preparation of a silk fibroin complex loaded with a polyhydroxy phenolic compound (β-SF-polyhydroxy phenolic compound)

**Step (1) Transformation of silk fibroin β-sheet conformation:** The silk fibroin (SF) was diluted 5 to 10 times with water, then slowly poured into an excessive amount of organic solvent that was vigorously stirred, and stirred for 2 to 4 h to complete the transformation of the β-sheet conformation. The liquid part was removed by mechanical separation methods such as suction filtration, centrifugation, and membrane filtration. The remaining part was repeatedly washed and then lyophilized to obtain β-sheet silk fibroin (β-SF), wherein the organic solvent was selected from methanol, ethanol, propylene glycol, butylene glycol, and the like, and preferably was ethanol; the mechanical separation method was preferably the high-speed centrifugation method, e.g., high-speed centrifugation at 10,000 rpm for 15 to 20 min.

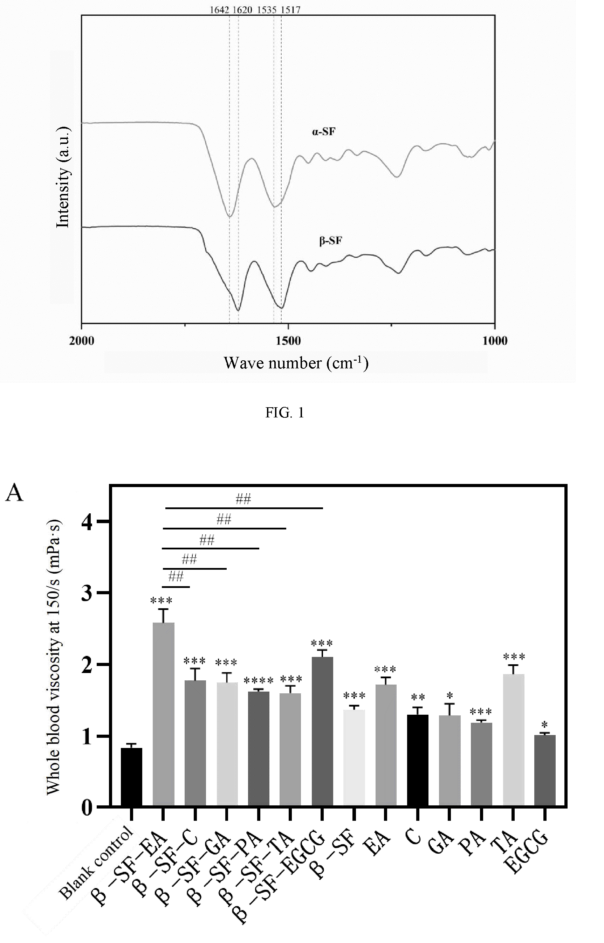

FTIR: 5% the sample and 95% KBr were mixed and ground into a fine powder, which was subjected to transmission scanning by a FTIR spectrometer (Nicolet is5) to obtain each spectrum in the transmission mode (scan 10 times) at a resolution of 4 cm⁻¹, and in a spectral range of 4000-500 cm⁻¹. The FTIR spectra of the β-sheet structure at a relatively high level showed sharp peaks at 1615-1640 cm⁻¹ and 1510-1525 cm⁻¹, while the FTIR spectra of SF in a natural state showed sharp peaks at 1640-1660 cm⁻¹ and 1535-1542 cm⁻¹, which corresponded to α-SF. As can be seen from the determination results in FIG. 1, the characteristic peaks of the natural α-SF were 1642 cm⁻¹ and 1535 cm⁻¹. The characteristic peaks of the SF treated in step (1) were 1620 cm⁻¹ and 1517 cm⁻¹. It can be seen that the SF has completed β-sheet conformation transformation and become β-SF.

**Step (2) Self-assembly of polyhydroxy phenolic compound and silk fibroin:** The β-SF obtained in step (1) was made into a suspension in water, and the suspension was stirred in a magnetic stirrer at 500-2000 rpm; the polyhydroxy phenolic compound was dissolved or suspended in a solvent. If it was water-soluble, water was used as a solvent; if insoluble, water or an organic solvent such as ethanol, propylene glycol, or butylene glycol was used as a solvent. The prepared solution or suspension was added dropwise to the β-SF suspension, and then mixed and stirred for 12 to 48 h to obtain a silk fibroin complex loaded with a polyhydroxy phenolic compound.

The polyhydroxy phenolic compound was selected from water-insoluble polyhydroxy phenolic compounds, including ellagic acid (EA), resveratrol (RES), quercetin (QUE), baicalein (BAE), kaempferol (KAE), lignan, curcumin (CUR), chlorogenic acid (CGA), ferulic acid (FA), caffeic acid (CFA), syringic acid (SA), sinapic acid, rutin (RUT), myricetin (MYR), magnolol acid (MA), fisetin (FIS), puerarin (PUE), rhein (RHE), lonicerin (Lon), proanthocyanidins (PC), p-coumaric acid (p-CA), vanillic acid (VA), hesperetin (Hes), naringenin (NAR), luteolin (Lut), genistein (Gen), emodin (Emo), oleanolic acid (Ola), danshensu (DS) and epigallocatechin gallate (EGCG); and water-soluble polyhydroxy phenolic compounds, including gallic acid (GA), pyrogallic acid (PA), catechin (C), tannic acid (TA), tea polyphenols (TP), acaciin, epigallocatechin (EGC), and the like.

### Embodiment 2: Preparation of a silk fibroin complex loaded with a polyhydroxy phenolic compound

Different from Embodiment 1, the organic solvent in step (1) was ethanol, and the mechanical separation method was high-speed centrifugation at 10,000 rpm for 15-20 min. In step (2), equal amounts of the polyhydroxy phenolic compound and the silk fibroin were added. In the preparation of the polyhydroxy phenolic compound suspension, the water-soluble polyhydroxy phenolic compound was prepared by using water as a solvent, while the insoluble polyhydroxy phenolic compound was prepared by using water or an organic solvent as a solvent. The mechanical separation method was high-speed or ultra-high-speed centrifugation to collect the precipitate. Step (3) was added: drying into powder: In step (2), the obtained solution was dried into powder by means of mechanical separation methods such as suction filtration, centrifugation, membrane filtration, and settlement, or was directly powdered using drying equipment such as a spray dryer, to obtain a silk fibroin complex loaded with a polyhydroxy phenolic compound; the mechanical separation method was preferably high-speed or ultra-high-speed centrifugation to collect the precipitate.

An insoluble polyhydroxy phenolic compound was used as a raw material: to prepare an ellagic acid-silk fibroin complex (β-SF-EA), a resveratrol-silk fibroin complex (β-SF-RES), a quercetin-silk fibroin complex (β-SF-QUE), a baicalein-silk fibroin complex (β-SF-BAE), a kaempferol-silk fibroin complex (β-SF-KAE), a lignan-silk fibroin complex (β-SF-Lignan), a curcumin-silk fibroin complex (β-SF-CUR), a chlorogenic acid-silk fibroin complex (β-SF-CGA), a ferulic acid-silk fibroin complex (β-SF-FA), a caffeic acid-silk fibroin complex (β-SF-CFA), a syringic acid-silk fibroin complex (β-SF-SA), a sinapic acid-silk fibroin complex (β-SF-Sinapic Acid), a rutin-silk fibroin complex (β-SF-RUT), a myricetin-silk fibroin complex (β-SF-MYR), a magnolol acid-silk fibroin complex (β-SF-MA), a fisetin-silk fibroin complex (β-SF-FIS), a puerarin-silk fibroin complex (β-SF-PUE), a rhein-silk fibroin complex (β-SF-RHE), a lonicerin-silk fibroin complex (β-SF-Lon), a proanthocyanidins-silk fibroin complex (β-SF-PC), a p-coumaric acid-silk fibroin complex (β-SF-p-CA), a vanillic acid-silk fibroin complex (β-SF-VA), a hesperetin-silk fibroin complex (β-SF-Hes), a naringenin-silk fibroin complex (β-SF-NAR), a luteolin-silk fibroin complex (β-SF-Lut), a genistein-silk fibroin complex (β-SF-Gen), an emodin-silk fibroin complex (β-SF-Emo), an oleanolic acid-silk fibroin complex (β-SF-Ola), a danshensu-silk fibroin complex (β-SF-DS) and an epigallocatechin gallate-silk fibroin complex (β-SF-EGCG); a water-soluble polyhydroxy phenolic compound is used as a raw material: to prepare a gallic acid-silk fibroin complex (β-SF-GA), a pyrogallic acid-silk fibroin complex (β-SF-PA), a catechin-silk fibroin complex (β-SF-C), a tannic acid-silk fibroin complex (β-SF-TA), an acaciin-silk fibroin complex (β-SF-Acaciin), an epigallocatechin-silk fibroin complex (β-SF-EGC) and a tea polyphenols-silk fibroin complex (β-SF-TP).

### Embodiment 3: Evaluation of coagulation effect

The obtained β-SF-polyhydroxy phenolic compound was made into a 2 mg/mL test solution using normal saline to evaluate its blood coagulation effect: Blood was collected from the abdominal aorta of New Zealand white rabbits and transferred into sodium heparin anticoagulant tubes. 2 mL of the test solution was added to each tube, with normal saline as the control. After thorough mixing, the mixture was incubated at 37 °C for 15 min, and whole blood viscosity was determined using an automatic blood rheometer (all operations were completed within 2 h after blood collection).

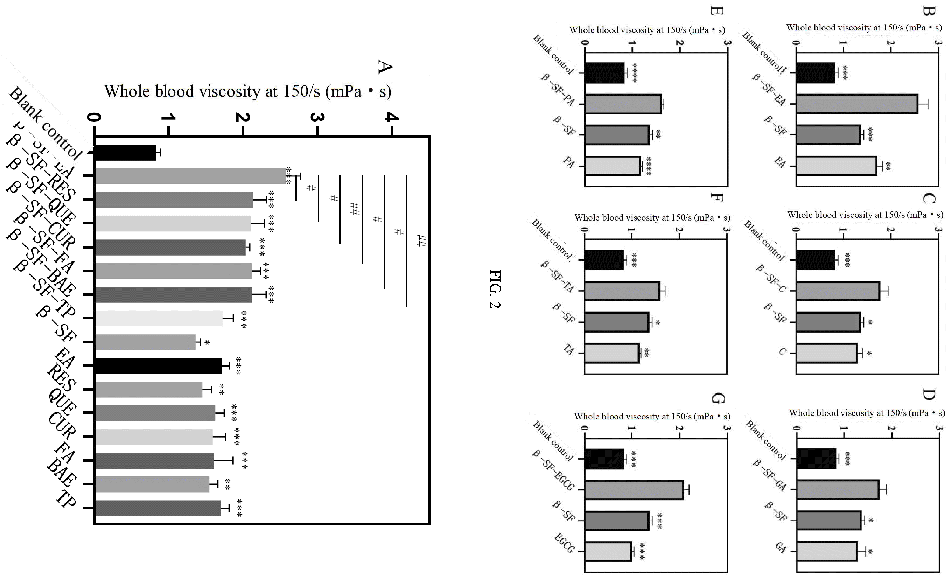

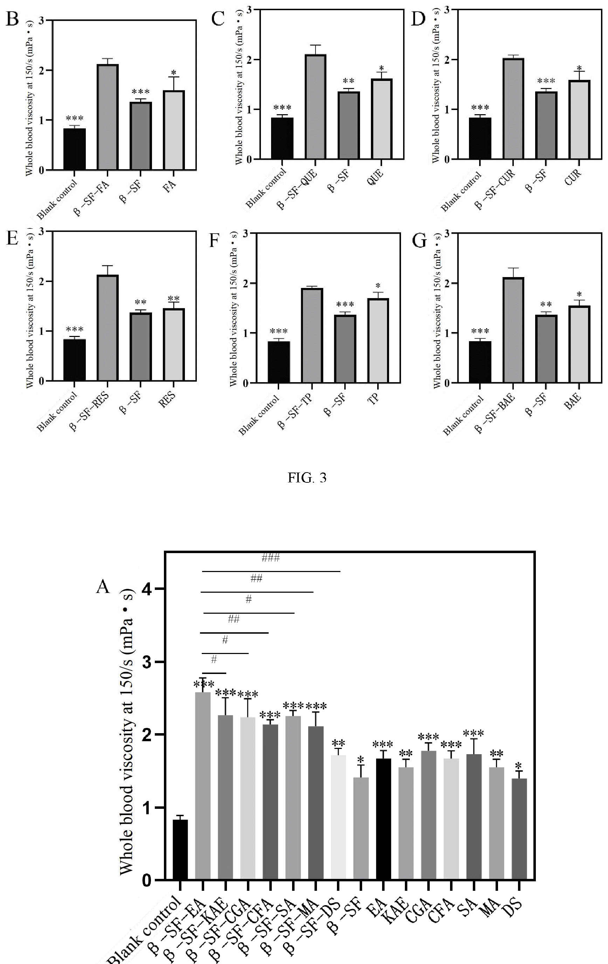

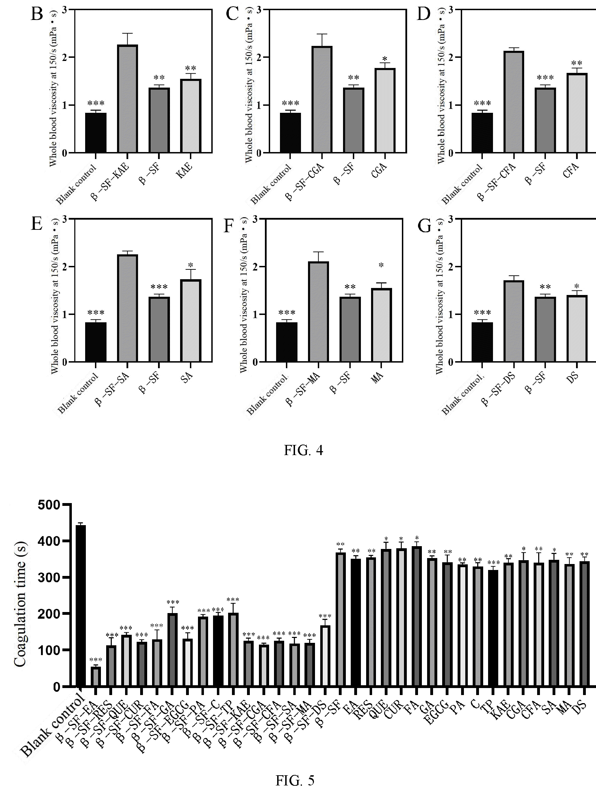

Whole blood viscosity is the result of friction between blood cells and plasma protein molecules when the blood is in motion. It is the most important indicator in hemorheology. An increase in blood viscosity indicates impaired blood flow, accompanied by reduced deformability and enhanced aggregation of red blood cells. This leads to a decreased blood flow volume and a reduced perfusion capacity within microvessels and capillaries. The results in FIG. 2 to FIG. 4 demonstrate that the silk fibroin complex loaded with a polyhydroxy phenolic compound (β-SF-polyhydroxy phenolic compound) could significantly elevate whole blood viscosity compared with the blank control group (P<0.05). Moreover, its performance was markedly superior to those of β-SF and the original phenolic compound alone (P<0.05), indicating that the formation of the complex could remarkably enhance the hemostatic efficacy; further, the complex formed by self-assembly of the silk fibroin and an insoluble polyhydroxy phenolic compound generally exhibited a superior effect compared with the complex formed by self-assembly of the silk fibroin and a water-soluble polyhydroxy phenolic compound.

0.1 g of the test drug powder was weighed and put in a 1.5 mL centrifuge tube. At the same time, 0.5 mL of anticoagulant was measured and added to the centrifuge tubes containing different drugs. The centrifuge tubes were vertically placed on the experimental workbench and timing was started. The centrifuge tubes were rotated and inverted 30s later and whether the blood was flowing was observed. This process was repeated until the anticoagulant could not flow any more. Timing was stopped and the time was recorded as the coagulation time. The results in FIG. 5 show a similar trend to those in FIG. 2 to FIG. 4.

Among all complexes, β-SF-EA exhibited an optimal coagulation effect, which might be attributed to the multiple hydrogen bonds and planar structure of EA. On the one hand, the presence of multiple hydrogen bonds enhances the synergistic effect among hydrogen bonds, further increasing the stability of hydrogen bonds. On the other hand, ellagic acid has a symmetrical catechol structure and a planar structure, which can form intramolecular and intermolecular hydrogen bonds, enhancing the rigidity of the complex molecule. Such stable molecular structures can reduce molecular dynamic fluctuations, thereby indirectly elevating the stability of intermolecular hydrogen bonds.

### Embodiment 4: Preparation of a polyhydroxy phenolic compound-silk fibroin complex (α-SF-polyhydroxy phenolic compound)

Different from Embodiment 2, step (1) was deleted, and β-SF in step (2) was replaced with untreated SF, i.e., natural SF with α-helix conformation. Equal amounts of the polyhydroxy phenolic compound and the silk fibroin were added in step (2) to prepare a polyhydroxy phenolic compound-α-silk fibroin complex, designated as α-SF-polyhydroxy phenolic compound.

### Embodiment 5: Preparation of a polyhydroxy phenolic compound-silk fibroin complex (SH-SF-polyhydroxy phenolic compound)

Different from Embodiment 2, the following method was adopted in step (1): the silk fibroin was dissolved in an aqueous solution containing tris(2-carboxyethyl)phosphine hydrochloride, and kept at room temperature to take reduction reaction for 5 min to obtain a reduced SF solution with a concentration of 1.0-5.0 wt%. The rest are the same as the methods described in Embodiment 2, where equal amounts of the polyhydroxy phenolic compound and the silk fibroin were added in step (2) to prepare a polyhydroxy phenolic compound-silk fibroin complex, designated as SH-SF-polyhydroxy phenolic compound. This method breaks disulfide bonds to expose hydrophobic amino acids, thereby enabling binding with polyhydroxy phenolic compounds such as ellagic acid.

### Embodiment 6: Evaluation of self-assembly efficiency

The polyhydroxy phenolic compound used ellagic acid (EA) as a model drug. Three types of ellagic acid-silk fibroin complexes, namely β-SF-EA, α-SF-EA and SH-SF-EA, were prepared following the steps described in Embodiments 2, 4 and 5, with water as a solvent for EA. The assembly efficiency was evaluated using drug loading (DL) and encapsulation efficiency (EE) according to Formula 1) and Formula 2) respectively, where the amount of EA in the "silk fibroin complex" was calculated by the difference method, that is, subtracting the free amount of EA after its co-incubation with SF from the total amount of EA added. The content of EA was analyzed by high performance liquid chromatography (HPLC), with the chromatographic column being a Hedera C18 column, the mobile phase being acetonitrile: water = 20:80 (v/v), a flow rate of 1.0 mL/min, a detection wavelength of 254 nm, a sampling size of 10 µL, and a chromatographic acquisition time of 15 min. DL and EE were calculated using the following formulae: $DL = Amount of EA in the silk fibroin complex / Total amount of SF and EA added$ Formula 1) $EE = Amount of EA in the silk fibroin complex / Amount of EA added$ Formula 2)

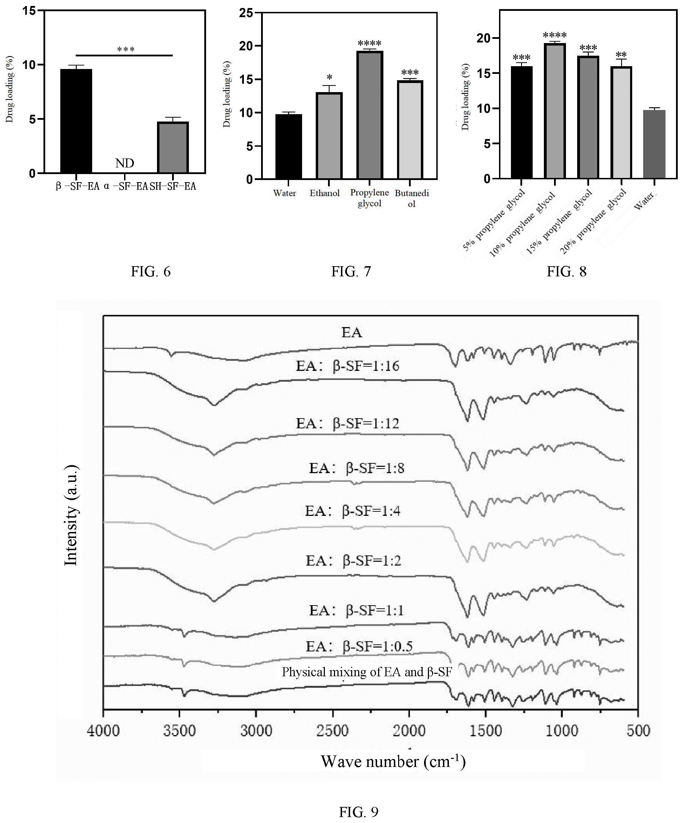

From the results in FIG. 6, it can be seen that DL: β-SF-EA> SH-SF-EA>>α-SF-EA, where the DL of β-SF-EA reached 9.6%, while EA was almost undetectable in α-SF-EA, indicating that the self-assembly efficiency of α-helix silk fibroin and ellagic acid is very low, highlighting the necessity of conformational inversion of the silk fibroin; SH-SF-EA exposed its hydrophobic amino acids by disrupting the disulfide bonds within the silk fibroin molecule, thereby achieving self-assembly with ellagic acid, but the DL was only 4.5%, much lower than that of β-SF-EA (P < 0.001).

### Embodiment 7: Evaluation of the DL of EA for solvents

The polyhydroxy phenolic compound used ellagic acid (EA) as a model drug, β-SF-EA was prepared following the steps described in Embodiment 2 and in step (2), a polyhydroxy phenolic compound suspension was prepared using water, ethanol, propylene glycol, butylene glycol or the like as a solvent. If an organic solvent was used as a suspension solvent, then after the polyhydroxy phenolic compound suspension was incubated with β-SF, the final concentration of the organic solvent in the resulting solution was 10%; their DLs were evaluated by the method described in Embodiment 6. The addition of organic solvents effectively improved the DL of EA, with propylene glycol > butylene glycol > ethanol > water in terms of DL, with propylene glycol achieving the highest DL, up to 19.57±1.01%.

Next, the effect of the concentration of propylene glycol on the DL of EA was evaluated. In step (2), propylene glycol was used as a solvent to prepare an EA suspension. After the suspension was incubated with β-SF, the final concentration of propylene glycol in the resulting solution was 0%, 5%, 10%, 15%, 20% respectively. The results in FIG. 8 show that the DL values in the propylene glycol aqueous solution groups were all superior to that in the water group. Among them, the 10% propylene glycol aqueous solution group had the best effect, with a DL value of 19.57±1.01%.

### Embodiment 8 Evaluation of different feeding ratios of EA:SF

The polyhydroxy phenolic compound used ellagic acid (EA) as a model drug, β-SF-EA was prepared following the steps described in Embodiment 2 and in step (2), an EA suspension was prepared using propylene glycol as a solvent. After the suspension was incubated with β-SF, the final concentration of propylene glycol in the resulting solution was 10%. Meanwhile, the feeding ratio of EA to β-SF was controlled at EA:β-SF=1:0.5, 1:1, 1:2, 1:4, 1:8, 1:12, 1:16 (w/w). From Table 1, it can be seen that when EA: β-SF=1:0.5, 1:1, the DL and EE of EA were almost unobservable, while they were observable in other groups; meanwhile, FTIR was performed to the samples in each group by the method described in Embodiment 1 (FIG. 9). In the groups of EA: β-SF=1:2, 1:4, 1:8, 1:12, 1:16 (w/w), disappearance of the hydroxyl peak (3471 cm⁻¹) of EA was observed, suggesting that EA and β-SF had bound with hydrogen bond and the self-assembly process was successful. However, the groups of EA: β-SF=1:0.5, 1:1 and their physical mixture groups still had the hydroxyl peak of EA, and could not achieve self-assembly of the two molecules. The above results collectively indicate that only when the feeding ratio EA: β-SF < 1:1 (w/w), can the self-assembly of β-SF and EA be achieved, and when EA: β-SF=1:4, EA can achieved maximum DL and EE (Table 1), and this ratio is referred to as the optimal feeding ratio.

Further, the effect of different EA: β-SF feeding ratios on whole blood viscosity was evaluated. The results in Table 2 indicate that compared with the normal saline group, each group with EA: β-SF<1:1 could significantly increase whole blood viscosity at low, medium, and high shear rates (P<0.5), which were higher than those of the EA and β-SF groups (P<0.5). This indicates that the β-SF-EA prepared with a feeding ratio of EA: β-SF<1:1 had a significant coagulation efficacy, and EA and β-SF synergistically enhanced each other's effect. The coagulation efficacy was the best when EA: β-SF ratio=1:4.

To sum up, β-SF-EA was prepared using β-sheet SF, with a feeding ratio of EA:β-SF<1:1 (w/w). The specific steps are shown in Embodiment 1; the optimal process is that EA:β-SF=1:4 (w/w), an EA suspension was prepared from EA by using propylene glycol as a solvent in step (2), and after co-incubation with SF, the final concentration of propylene glycol in the resulting solution was 10%. Self-assembly of EA and β-SF could not be achieved without β-sheet conformation transformation of SF or when the feeding ratio of EA:β-SF≥1:1. When EA:β-SF=1:2 (w/w), DL and EE were low, indicating that although the self-assembly of EA and β-SF could be achieved in this case, a significant loss occurred.

**Table 1 Effects of the Feeding Ratio of β-SF to EA on DL and EE (n=3)**

| Group | EA: β-SF (w/w) | DL (%) | EE (%) |
|---|---|---|---|
| 1 | 1:0.5 | ND | ND |
| 2 | 1:1 | ND | ND |
| 3 | 1:2 | 15.88±1.03 | 45.96±0.99 |
| 4 | 1:4 | 19.57±1.01 | 97.88±0.56 |
| 5 | 1:8 | 10.6±1.12 | 95.45±0.45 |
| 6 | 1:12 | 6.95±0.99 | 90.08±0.65 |
| 7 | 1:16 | 2.76±0.98 | 90.37±0.64 |

| | | | |
|---|---|---|---|
| ND represents Not Detected. | | | |

**Table 2 Effects of EA: β-SF Feeding Ratios on Whole Blood Viscosity (n=3)**

| Group | EA: β-SF (w/w ) | Whole blood viscosity/mPa·s | | | | |
|---|---|---|---|---|---|---|
| | | 1/s | 30/s | 100/s | 150/s | 200/s |
| Normal saline group | | 0.810±0.023^{####} | 0.804±0.024^{####} | 0.801±0.056^{####} | 0.851±0.064^{####} | 0.855±0.045^{####} |
| β-S F group | | 1.311±0.142**^{, ####} | 1.318±0.159**^{, ####} | 1.317±0.145**^{, ####} | 1.311±0.222*^{, ####} | 1.320±0.111**^{, ####} |
| EA group | | 1.755±0.123***^{, ####} | 1.742±0.236**^{, ###} | 1.658±0.122***^{, ###} | 1.75±0.146***^{, ####} | 1.800±0.255**^{, ####} |
| 1 | 1:0.5 | 1.611±0.361*^{,##} | 1.594±0.389*^{, ###} | 1.601±0.332*^{, ###} | 1.655±0.288**^{, ###} | 1.676±0.243**^{, ###} |
| 2 | 1:1 | 1.625±0.458*^{,##} | 1.651±0.318*^{, ###} | 1.624±0.361*^{, ###} | 1.75±0.274**^{, ###} | 1.688±0.250**^{, ###} |
| 3 | 1:2 | 2.911±0.127*** *, ^{##} | 2.890±0.170*** *^{,##} | 2.940±0.481**^{,#} | 2.977±0.178*** *^{,##} | 2.870±0.192*** *^{,##} |
| 4 | 1:4 | 3.635±0.078*** * | 3.800±0.198*** * | 3.940±0.246*** * | 3.963±0.118*** * | 3.950±0.132*** * |
| 5 | 1:8 | 2.960±0.071*** *^{, ###} | 3.170±0.356***^{,} ns | 3.093±0.181*** *^{, ##} | 3.205±0.148*** *^{, ##} | 3.095±0.134*** *^{,##} |
| 6 | 1:12 | 2.885±0.445**^{,#} | 2.960±0.198*** *^{, ##} | 2.623±0.261***^{, ##} | 2.750±0.212***^{, ###} | 2.845±0.078*** *^{, ###} |
| 7 | 1:16 | 2.747±0.403**^{,#} | 2.670±0.156*** *^{,##} | 2.673±0.103*** *^{, ##} | 2.650±0.100*** *^{, ###} | 2.515±0.021 *** *^{, ####} |

| | | | | | | |
|---|---|---|---|---|---|---|
| *: Compared with the normal saline group, **P<0.05, **P<0.01, ***P<0.001;* #: compared with the EA: β-SF=1:4 group, *#P* <0.05, *##P<0.01, ###P<0.001.* | | | | | | |

### Embodiment 9: Molecular docking of silk fibroin complex

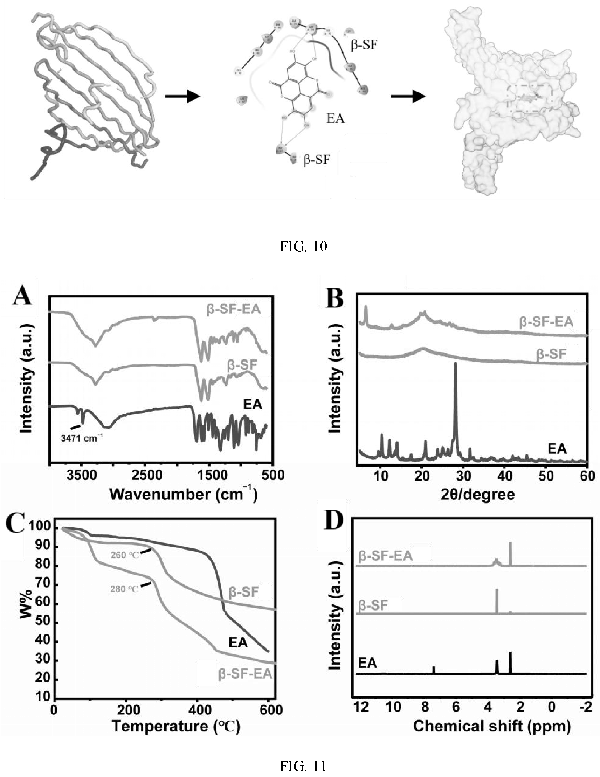

A computer simulation study was conducted on the binding mode of ellagic acid and silk fibroin. The crystal structure of the heavy chain of the silk fibroin (PDB number: 3UA0) was obtained from the PDB bank. Given the limited previous studies on binding modes, the SiteMap module in Schrödinger 2018 was adopted to rationally predict potential binding sites. Hydrogen atoms were added to the crystal structure of the silk fibroin, and irrelevant ions were eliminated. After that, the small molecule of EA was converted into a three-dimensional conformation via the LigPrep module, up to 32 isomers were output and in the end, 20 binding poses were output via Glide extra precision (XP). As shown in FIG. 10, after the two phenolic hydroxyl groups in the small molecule of EA form hydrogen bonds with L74, additional hydrogen bonds need to be formed with D27 on another heavy chain. This indicates that stable binding can be achieved only by further forming hydrogen bonds with another two highly symmetrical hydroxyl groups in EA. Further, since the heavy chains in the silk fibroin maintain a highly similar β-sheet secondary structure, the small molecule of EA may not have sufficient hydrophobic interaction with the two heavy chain proteins to maintain stability. A reasonable guess is that the small molecules of EA will at least bind and encapsulate three to four heavy chain proteins to achieve stable binding. The molecular docking results explain the molecular mechanism of the experimental result in Embodiment 8, "EA: β-SF<1:1 (w/w) is required for the self-assembly of β-SF and EA".

The samples in Embodiments 10-14 and 22-24 are β-SF-EA prepared using the optimal process.

### Embodiment 10: Representation of a silk fibroin complex loaded with a polyhydroxy phenolic compound

FT-IR confirmed the self-assembly of EA and β-SF (FIG. 11A): The disappearance of the hydroxyl peak (3471 cm⁻¹) of EA indicates that EA and β-SF may be self-assembled through intermolecular hydrogen bonds. The weakening or disappearance of the characteristic peak of EA after bound with silk fibroin in X-ray diffraction (XRD) (FIG. 11B) indicates that it is not a simple physical mixture but a new crystal phase formed through interaction, confirming the results of FT-IR; at the same time, the change in crystal form was observed and β-SF showed broad diffraction peaks, indicating that they were amorphous macromolecules; EA exhibited many strong diffraction peaks, indicating that it was in a crystalline state. After the self-assembly of β-SF and EA, the intensity of the EA peak significantly decreased, from which it could be determined that β-SF-EA was primarily an amorphous macromolecule.

The thermogravimetric (TG) curve was evaluated and divided into three stages (FIG. 11C). The first stage was from room temperature to 120°C and the weight loss was approximately 6.1%, mainly from the evaporation and desorption of adsorbed water; in the second stage, the weight loss occurred between 150°C and 450°C, corresponding to the breakdown of the molecular chains of the silk fibroin, the breakdown of the side chains of the amino acid residues, and the cleavage of the peptide bonds, wherein the silk I crystalline structure (α-helix and random coil) degraded at approximately 250 °C, while the silk II crystalline structure (β-sheet) degraded at approximately 260 °C; β-SF exhibited a main degradation peak at 260°C, and a stable β-sheet conformation was formed on the surface; interestingly, the main degradation peak of β-SF-EA shifted to 280°C, indicating that the silk II structure is more stable. This might be attributed to the strong interaction between EA and the protein during the self-assembly process, thereby enhancing the stability of the complex. In order to determine the optimal amount of EA doped to β-SF, proton nuclear magnetic resonance (1H NMR) (FIG. 11D) was conducted: In β-SF-EA, the peaks related to EA were significantly weakened (<0.007), confirming that EA bound to β-SF by means of the phenolic hydroxyl group, further demonstrating their binding.

### Embodiment 11: Evaluation of coagulation effect

### (1) Treatment of plasma samples

New Zealand white rabbits were acclimatized for one week and fasted overnight prior to the experiment. The rabbits were anesthetized via intraperitoneal injection of 1% sodium pentobarbital at a dosage of 3 mL·kg⁻¹. Blood was collected from the abdominal aorta into 5 mL sodium heparin anticoagulant tubes after anesthesia. After blood collection, the blood was thoroughly mixed with the anticoagulant and reserved for subsequent use.

### (2) In vitro blood adsorption capacity test

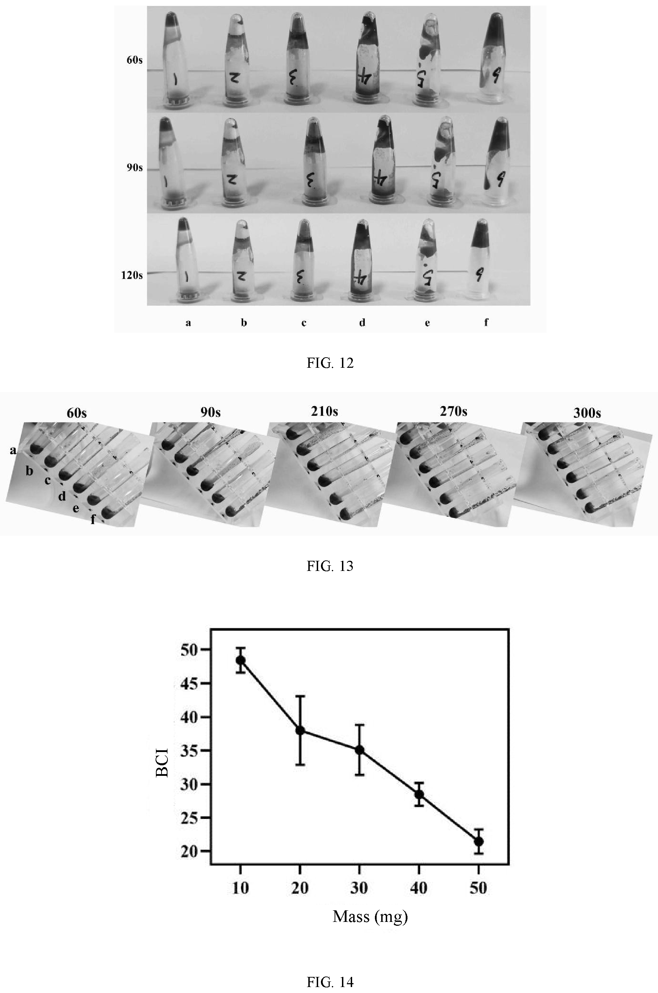

0.1 g of the test drug powder was weighed and put in a 1.5 mL centrifuge tube. At the same time, 0.5 mL of anticoagulant was measured and added to the centrifuge tubes containing different drugs. They were placed vertically on the experimental workbench and timing was started. The centrifuge tubes were rotated and inverted 30s later and whether the blood was flowing was observed. This process was repeated until the anticoagulant could not flow any more. Timing was stopped. The results in FIG. 12 show that within two minutes, β-SF-EA achieved complete adsorption of the blood, while other groups failed to achieve this effect. This indicates that the blood adsorption ability of β-SF-EA is significantly superior to the positive controls Yunnan Baiyao and tranexamic acid, and also significantly superior to β-SF and EA alone, suggesting that β-SF and EA can synergistically enhance the effect.

### (3) Tube tilting coagulation experiment

5.0 mg of the test drug powder was precisely weighed and put in a 10 mL test tube by paving it at the bottom of the test tube as far as possible. 1 mL of anticoagulant was measured and added to the test tube, 25 µL of 0.2 mol·L⁻¹ CaCl₂ solution was added further, they were thoroughly mixed at once and timing was started immediately. The test tube stood for 1 min and was tilted every 30 s. Timing was stopped when the blood was coagulated and stopped flowing. FIG. 13 shows that the complete coagulation time of the blood in the β-SF-EA group (2 min) was significantly shorter than those in the Yunnan Baiyao group (5 min), the tranexamic acid group (4 min 30 s), the β-SF group (5 min), and the EA group (>5 min), indicating that β-SF and EA could synergistically enhance the effect. This experiment shows that β-SF-EA exhibited faster coagulation compared to other drugs.

### (4) Determination of hemorheology

Whole blood viscosity is the result of friction between blood cells and plasma protein molecules when the blood is in motion. It is the most important indicator in hemorheology. An increase in blood viscosity indicates impaired blood flow, accompanied by reduced deformability and enhanced aggregation of red blood cells. This leads to a decreased blood flow volume and a reduced perfusion capacity within microvessels and capillaries. The whole blood viscosity was measured at shear rates of 1·s⁻¹, 30·s⁻¹, 100·s⁻¹, 150·s⁻¹, 200·s⁻¹ by the method described in Embodiment 3 after the blood was treated with β-SF-EA. The results in Table 3 show that β-SF-EA could significantly elevate whole blood viscosity (P < 0.05) regardless of the shear rate and was markedly superior to the commercially available chitosan hemostatic powder group (P<0.05). The commercially available chitosan hemostatic powder used in the control group was instant composite microporous polysaccharide hemostatic powder purchased from Success Bio-Tech Co., Ltd., with chitosan as its main active ingredient.

**Table 3 Comparison between β-SF-EA and Chitosan in Terms of Whole Blood Viscosity (n=3)**

| | 1/s (mPa·s) | 30/s (mPa·s) | 100/s (mPa·s) | 150/s (mPa·s) | 200/s (mPa·s) |
|---|---|---|---|---|---|
| Blank control | 0.855±0.011 | 0.854±0.021 | 0.902±0.010 | 0.911±0.013 | 0.910±0.013 |
| Chitosan hemostatic powder | 3.265±0.275^{***} | 3.185±0.007^{***} | 2.930±0.061^{***} | 2.973±0.087^{***} | 2.993±0.081^{***} |
| β-SF-EA | 3.635±0.118^{***,#} | 3.800±0.198^{***,##} | 3.940±0.246^{***,##} | 3.963±0.118^{***,##} | 3.950±0.132^{***,#} |

| | | | | | |
|---|---|---|---|---|---|
| *: Compared with the blank control group, **P*<0.05, ***P*<0.01, ****P*<0.001; #: compared with the commercially available chitosan hemostatic powder group, #*P* <0.05, ##*P*<0.01, ###*P*<0.001. | | | | | |

### (4) Blood coagulation index (BCI)

10, 20, 30, 40, 50 mg of β-SF-EA were prepared and flatly placed in several 50 mL centrifuge tubes, 0.1 mL of anticoagulant was gently added dropwise on each sample, then 0.02 mL of 0.2 mol/L CaCl₂ solution was added immediately, 25 mL of deionized water was added to the beaker 5 min later, the mixture was centrifuged at 300 rpm for 5 min, then the solution was removed and its Abs value was measured using an microplate reader at a wavelength of 540 nm. A control was set: by adding 0.1 mL of anticoagulant to the beaker and then adding 25 mL of deionized water. The Abs value measured at the same wavelength was assumed to be 100 as the reference value. Then, the BCI was calculated as: BCI = 100 × Aₛₐₘₚₗₑ / A_{control}. BCI is an indicator reflecting the blood coagulation effect. The smaller the BCI, the better the blood coagulation effect. FIG. 14 shows that increasing the dosage (increasing the concentration in the same volume) would significantly reduce BCI, showing a positive correlation between the coagulation effect and the dosage.

### Embodiment 12: Evaluation of hemostatic effect

Male SD rats (weighing approximately 250.0 g) were used to evaluate the hemostatic ability of β-SF-EA.

Rat tail amputation hemostasis experiment: SD rats were anesthetized via intraperitoneal injection of 1% pentobarbital sodium. A 6 cm long section was measured from the tail end of the rat, and cut off with surgical scissors. After bleeding, 20 mg of sample powder (β-SF-EA, Yunnan Baiyao, tranexamic acid) was sprinkled on the wound immediately. Timing was started from the moment of bleeding.

Rat liver wound hemostasis experiment: SD rats were anesthetized via intraperitoneal injection of 1% pentobarbital sodium. The anesthetized rats were fixed in a supine position on an anatomical table. A longitudinal incision was made along the ventral midline to open the abdominal cavity and expose the right hepatic lobe of the rat. The peritoneal fluid was blotted dry with clean gauze. A parallel wound approximately 2.0 cm in length and 0.5 cm in depth was created on the right hepatic lobe using a surgical blade. After bleeding from the wound, pre-weighed cotton was used to absorb the blood immediately. Subsequently, 20 mg of sample powder (EA, β-SF, β-SF-EA, Yunnan Baiyao, tranexamic acid) was sprinkled onto the wound surface, and timing was initiated simultaneously. The wound was compressed with cotton for 30 s, after which bleeding was observed. If oozing persisted, the wound was compressed with cotton for another 30 s followed by re-observation. This procedure was repeated until no obvious blood stains were visible on the cotton, indicating successful hemostasis. Timing was then terminated, and the hemostasis time was recorded.

Rat thigh muscle wound hemostasis experiment: SD rats were anesthetized via intraperitoneal injection of 1% pentobarbital sodium. The anesthetized rats were fixed in a supine position on an anatomical table. The leg hair was removed using a rat hair clipper. The inner side of the thigh was disinfected with alcohol, and then a surgical scalpel was used to make a muscle wound approximately 3 cm long and 1 cm deep. After bleeding from the wound, timing was started. 5 s after bleeding, 20 mg of sample powder (EA, β-SF, β-SF-EA, Yunnan Baiyao, tranexamic acid) was sprinkled on the wound surface, the hemostatic effect was observed and the hemostasis time was recorded.

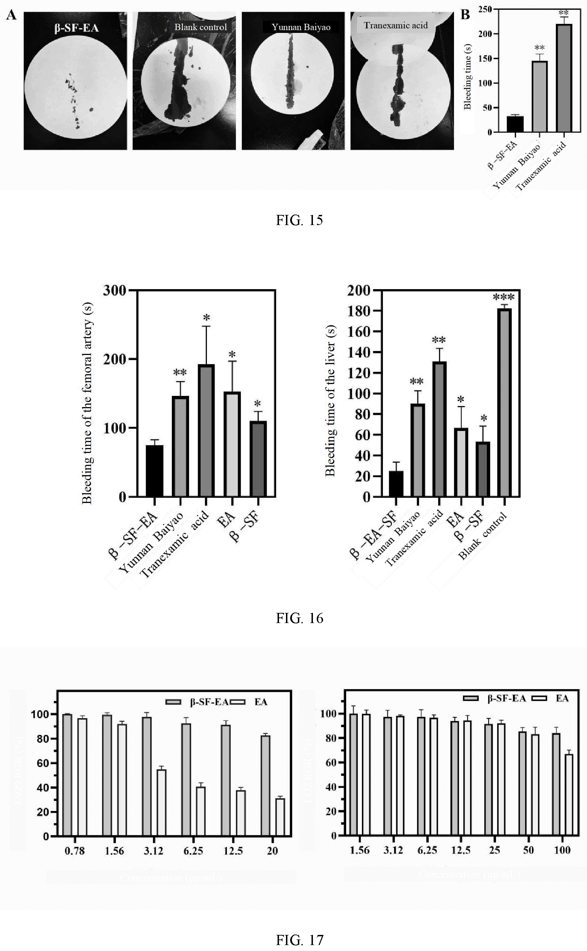

FIG. 15 is a diagram for rat tail amputation experiment. When β-SF-EA was applied to the wound, blood loss ceased rapidly with barely any blood residue left on the filter paper. It could significantly accelerate hemostasis within 30 s; the bleeding time of the blank control group exceeded five minutes, rendering the data statistically insignificant; thus, these data were not presented in FIG. B. As a comparison, the tranexamic acid group and the Yunnan Baiyao group took more than four times the time to stop bleeding, and the amount of bleeding was significantly higher than that of the β-SF-EA group (P<0.05).

FIG. 16 shows charts for bleeding time of liver and femoral artery in rats. The femoral artery bleeding time in the blank control group exceeded five minutes, making the data statistically insignificant; therefore, these data were not presented in the figure. FIG. 16 shows that β-SF-EA could significantly shorten the hemostasis time in both models. Its hemostatic efficacy was markedly superior to Yunnan Baiyao (P<0.05) and tranexamic acid (P<0.05) as well as the EA group (P<0.05) and the β-SF group (P<0.05). This demonstrates that silk fibroin and ellagic acid exert a synergistic hemostatic effect. The above results are consistent with the results of the hemorheology experiment and the tube tilting coagulation experiment in Embodiment 11. It was observed in both rat bleeding models that β-SF-EA had a higher blood settlement rate and could rapidly settle to a bleeding site compared to other drugs, while the powders of Yunnan Baiyao, tranexamic acid and β-SF were lighter and more likely to float on the surface of the whole blood and be washed away by the blood flow, thereby affecting their hemostatic effect.

### Embodiment 13: Safety evaluation

### (1) Cell resuscitation

The cryopreserved LO2 cells and L929 cells were taken out and gently shaken in a 37°C water bath until completely thawed. In the ultra-clean bench, the cell suspension in the cryogenic tube was transferred to the cell culture flask, and 4 mL of DMEM medium containing 10% fetal bovine serum was added to the culture flask. The mixture was gently pipetted to evenly disperse the cells in the medium, and then, placed in a 5% CO₂, 37°C cell incubator for culturing. After culturing for 6 h, the culture flask was disinfected with alcohol and put into the ultra-clean bench. The old culture medium was sucked away. The flask was washed, then 4 mL of culture medium was added, and the liquid was put into the cell incubator for culturing.

### (2) CCK8 quantitative method

0.2 mg of β-SF-EA and 0.2 mg of EA were weighed and dissolved in 1 mL of DMSO respectively. Gradient concentrations were set up for each of them.

The cells with logarithmic growth cycle were taken, and washed with a PBS solution. Then a trypsin digestion solution was added to make the cells in a suspended state. The cell density was calculated using a hemocytometer, then the cells were inoculated at 1×10⁵ cells/mL of cells into a 96-well plate per well, put into a cell incubator and cultured for 24 h. After cell attachment, the culture medium was sucked out, and the cells were washed with PBS twice. 100 µL of β-SF-EA extract and 100 µL of EA extract for the experiment groups, 100 µL of DMEM medium containing 10% fetal bovine serum for the blank control group, and 100 µL of paclitaxel extract for the positive control group were measured and added to cell-seeded 96-well plates respectively. Six parallel samples were set for each group, and put into a 37 °C, 5% CO₂ cell incubator and cultured for 48 h. The culture medium was sucked out, and the cells were washed with PBS twice. 100 µL of CCK8 solution (90 µL of DMEM medium + 10 µL of CCK8) was added. The cells were incubated in the incubator in darkness for 30 min, and then the absorbance (OD) was measured at 450 nm using a microplate reader. Six parallel samples were used at each time point, and the relative growth rate (RGR) of the cells was calculated according to the following formula: RGR %=OD _{experiment group}/OD _{blank control group}*100%.

The cytotoxicity grade was determined in accordance with the criteria shown in Table 4, and the cytotoxicity grade of positive controls shall not be lower than Grade 3. Samples with a cytotoxicity grade 0 or 1 were considered qualified.

**Table 4 Grading Criteria for Cell Growth**

| RGR/% | ≥100 | 75-99 | 50-74 | 25-49 | 1-24 |
|---|---|---|---|---|---|
| Grade | 0 | 1 | 2 | 3 | 4 |

We studied the toxicity of β-SF-EA and EA toward L929 cells and LO2 cells through the CCK-8 experiment. As shown in FIG. 17, the relative growth rates (RGR) in all β-SF-EA groups exceeded 85%. According to the cytotoxicity grading criteria specified in ISO 10993-1, β-SF-EA exhibited Grade 0 to Grade 1 cytotoxicity. Meanwhile, the addition of silk fibroin effectively enhanced the biocompatibility of ellagic acid. In conclusion, β-SF-EA does not affect cell growth and has desirable cell compatibility.

### (2) Hemolysis test

8 mL of fresh rabbit blood was diluted with 10 mL of normal saline. 20 mg of β-SF-EA was added to a 50 mL centrifuge tube, 10 mL of normal saline was added, the mixture was kept in a 37 °C water bath for 10 min, then 0.2 mL of the diluted rabbit blood was added, and they were gently shaken, incubated for 60 min and then centrifuged at 1000 rpm for 5 min. The supernatant was collected and its absorbance was measured at 540 nm. The positive control group was prepared by adding 0.2 mL of rabbit blood to 10 mL of distilled water, while the negative control group was prepared by adding 0.2 mL of rabbit blood to 10 mL of normal saline. The operation procedures were the same. Three parallel samples were prepared for each group. The hemolysis rate was calculated using the following formula: Hemolysis rate (%)=(Absorbance of the test sample - Absorbance of the negative control)/(Absorbance of the positive control - Absorbance of the negative control)*100%.

Direct contact between different materials and blood may cause rupture of red blood cells, resulting in hemolysis. The hemolysis test evaluated the degree of hemolysis in the material by measuring the concentration of hemoglobin. The calculated hemolysis rate of β-SF-EA was 1.13±0.02% (Table 5). In accordance with GB/T 4233.2, materials with a hemolysis rate below 5% are deemed qualified. Therefore, the prepared β-SF-EA meets the relevant international standards.

**Table 5 Hemolysis Rate (n=3)**

| S/N | Absorbance | | | Hemolysis rate |
|---|---|---|---|---|
| | β-SF-EA | Positive control | Negative control | |
| 1 | 0.030 | 0.901 | 0.020 | 1.13% |
| 2 | 0.031 | 0.922 | 0.021 | 1.11% |
| 3 | 0.031 | 0.886 | 0.021 | 1.15% |

### Embodiment 14: Evaluation of degradability

A certain mass of β-SF-EA was weighed, put in a PBS solution containing 0.1 µ/mL protease XIV, and incubated at 37 °C, with a PBS solution not containing protease XIV as a blank control sample. The samples (n=3) were incubated in a PBS solution and a PBS solution containing 0.1 µ/mL protease XIV for 5, 10, 15, 20, 25, and 30 days. For all the degraded samples, the solutions were replaced with fresh solutions at the fixed time every day. The degradation products were dried at 60 °C until the weight remained unchanged. The calculation formula for mass retention rate R_{M} is as follows: R_{M}/%=M_{dt}/Mᵢ*100%, where Mᵢ is initial mass, and M_{dt} is the mass retained after t days, mg.

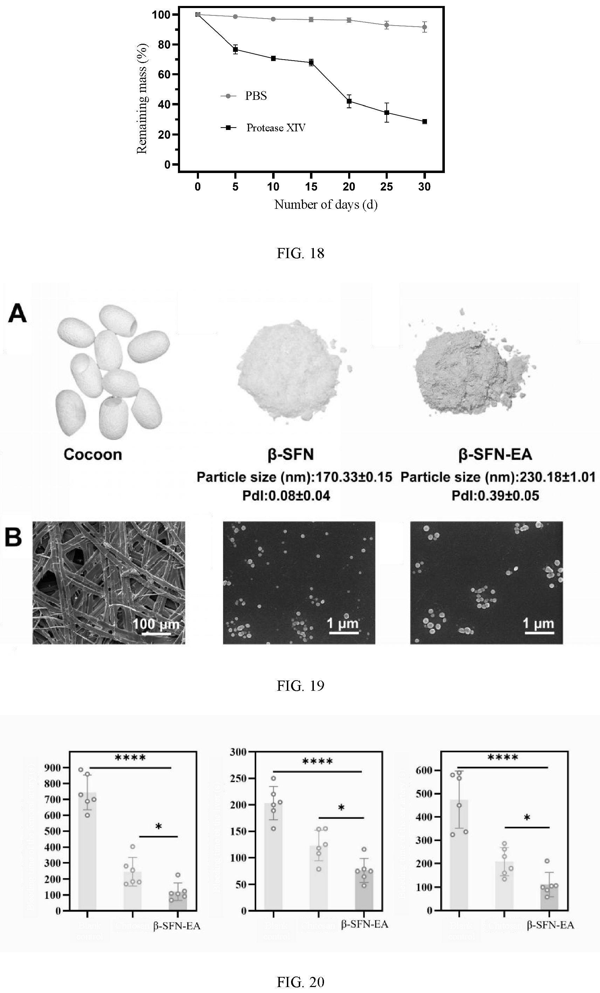

As hemostatic formulations intended for in vitro and in vivo applications, they shall have favorable biodegradability. Ideally, at the early stage of wound healing, the hemostatic powder can firmly adhere tissues together to prevent wound dehiscence. It is preferred to degrade slowly; with the healing of the wound, the formulation is degraded gradually, and the residual drug components can be fully degraded after wound closure, followed by absorption by the human body or excretion via metabolism. The degradation effect of β-SF-EA at 37 °C is shown in FIG. 18. It can be found that β-SF-EA exhibited a slow degradation rate in PBS, with a residual mass of 90.11±3.51% on day 30. In the PBS solution containing protease XIV, the degradation effect of β-SF-EA can be roughly divided into two stages: the first stage (0 - 15 days), in which the degradation rate was relatively small, and the remaining mass on day 15 was 67.83±2.25%; the second stage (15-30 d), in which the degradation rate began to increase significantly, and the remaining mass on day 30 was 24.50±0.70%, indicating that a large portion of it had been degraded, suggesting that it has good degradability.

### Embodiment 15: Preparation of silk fibroin nano-particles loaded with a polyhydroxy phenolic compound (β-SFN-polyhydroxy phenolic compound)

On the basis of Embodiment 1 or Embodiment 2, the silk fibroin complex loaded with a polyhydroxy phenolic compound was nanonized by chemical methods (desolvation, salting-out, microemulsion method, etc.), instrumental methods (electrospraying, electric field, supercritical fluid technology, etc.) and other methods (polymer blending, pH modification, nanoimprint lithography) to obtain silk fibroin nano-particles loaded with a polyhydroxy phenolic compound (β-SFN-polyhydroxy phenolic compound). Thus, the dosage forms of the formulation with a hemostatic efficacy, including gauze, sponge, spun fiber, spray, powder, granule, gel, sealant, ointment, film, patch and suppository, can be prepared.

### Embodiment 16: Preparation of hemostatic powder containing silk fibroin nano-particles loaded with ellagic acid

Silk fibroin was diluted with water 5 to 10 times and then slowly poured into an excessive amount of organic solvent that was vigorously stirred. The mixture was stirred for 2-4 h to complete the transformation of the β-sheet conformation. The liquid part was removed by suction filtration, high-speed centrifugation (high-speed centrifugation at 10,000 rpm for 15 to 20 min), and dialysis. The remaining part was repeatedly washed and then lyophilized to obtain β-sheet silk fibroin. The obtained β-SF was dispersed in water to obtain a suspension and stirred with a magnetic stirrer at 500-2,000 rpm, the polyphenol was suspended in solvents such as water, ethanol, propylene glycol and butanediol, the polyphenol suspension was added dropwise into the β-SF suspension, the mixture was stirred for 12-48 h, the resulting solution was subjected to mechanical separation methods such as suction filtration, centrifugation and membrane filtration to remove the liquid part, the remaining part was lyophilized using a lyophilizer, and the lyophilized solid was then ground and crushed, and then sieved to obtain a hemostatic powder.

### Embodiment 17: Preparation of a hemostatic spray containing silk fibroin nanoparticles loaded with ellagic acid

Silk fibroin was diluted with water 5 to 10 times and then slowly poured into an excessive amount of organic solvent that was vigorously stirred. The mixture was stirred for 2-4 h to complete the transformation of the β-sheet conformation. The liquid part was removed by suction filtration, high-speed centrifugation (high-speed centrifugation at 10,000 rpm for 15 to 20 min), and dialysis. The remaining part was repeatedly washed and then lyophilized to obtain β-sheet silk fibroin. The obtained β-SF was dispersed in water to obtain a suspension and stirred with a magnetic stirrer at 500-2,000 rpm, the polyphenol was suspended in solvents such as water, ethanol, propylene glycol and butanediol, the polyphenol suspension was added dropwise into the β-SF suspension, the mixture was stirred for 12-48 h, the resulting solution was subjected to mechanical separation methods such as suction filtration, centrifugation and membrane filtration to remove the liquid part, the remaining part was dissolved in water to get a 30%w/w suspension, the suspension was injected into a pressure-resistant lightweight handheld spray bottle, and the bottle was shaken up for 10 s to obtain a hemostatic spray.

### Embodiment 18: Preparation of hemostatic granules containing silk fibroin nanoparticles loaded with ellagic acid

Silk fibroin was diluted with water 5 to 10 times and then slowly poured into an excessive amount of organic solvent that was vigorously stirred. The mixture was stirred for 2-4 h to complete the transformation of the β-sheet conformation. The liquid part was removed by suction filtration, high-speed centrifugation (high-speed centrifugation at 10,000 rpm for 15 to 20 min), and dialysis. The remaining part was repeatedly washed and then lyophilized to obtain β-sheet silk fibroin. The obtained β-SF was dispersed in water to obtain a suspension and stirred with a magnetic stirrer at 500-2,000 rpm, the polyphenol was suspended in solvents such as water, ethanol, propylene glycol and butanediol, the polyphenol suspension was added dropwise into the β-SF suspension, the mixture was stirred for 12-48 h, the resulting solution was subjected to mechanical separation methods such as suction filtration, centrifugation and membrane filtration to remove the liquid part, and the remaining part was lyophilized using a lyophilizer to obtain aggregated silk fibroin particles (β-SF-polyphenol). Finally, depending on the requirements, the lyophilized particles were subjected to grinding, sieving and other treatments to obtain hemostatic granules in the desired particle size.

Embodiments 19-22 used EA as a model drug, and β-SFN-EA powder was prepared by the method described in Embodiment 16: A silk fibroin complex (β-SF-EA) was prepared under the optimal process, and then a nanonization process was added to obtain β-SFN-EA. The optimal process is that EA:β-SF=1:4 (w/w), an ellagic acid suspension was prepared from ellagic acid by using propylene glycol as a solvent in step (2), and after co-incubation with SF, the final concentration of propylene glycol in the resulting solution was 10%. In the same time, β-SFN not loaded with EA was used as a control.

### Embodiment 19: Presentation of hemostatic powder containing silk fibroin nano-particles loaded with ellagic acid (β-SFN-EA)

FIG. 19A-C show the macroscopic morphology of cocoon, β-SFN and β-SFN-EA. The morphological examination was conducted using a TESCAN MAIA 3 GMU field emission scanning electron microscope. The scanning electron microscope (SEM) images in FIG. 19D-F revealed that both β-SF and β-SF-EA exhibited quasi-spherical morphology, and enlarged volume and particle size were observed for β-SF-EA. Particle sizes were measured by NANO ZS90 through dynamic light scattering (DLS). The results in Table 6 show that the average particle size of β-SFN-EA (201.18 ± 2.17 nm) was larger than that of β-SFN (170.33 ± 0.15 nm). Both groups of samples had a relatively large ζ potential (<-20 mV), indicating that β-SFN-EA tends to remain stable in the aqueous solution due to electrostatic repulsion. In the β-SFN group, the polydispersity index (PdI) was less than 0.1, indicating a uniform particle size distribution, and in the β-SFN-EA group, the PdI increased to 0.39±0.05, indicating a decrease in particle size uniformity after preparation. Meanwhile, it was observed that β-SFN was light and tended to float in solution, whereas the prepared β-SFN-EA was heavy and readily settled in the solution.

**Table 6 Particle Size, PdI and ζ Potential (n=3)**

| S/N | Name | Particle size/nm | PdI | ζ potential/mV |
|---|---|---|---|---|
| 1 | β-SF-EA | 201.18±2.17 | 0.39±0.05 | -20.1±0.81 |
| 2 | β-SF | 170.33±0.15 | 0.082±0.04 | -22.3±0.62 |

### Embodiment 20: Evaluation of hemostatic effect

New Zealand white rabbits (female, 2.5-3.0 kg) were fixed on an iron plate for surgery and anesthetized via intraperitoneal injection of 3% sodium pentobarbital. The positive control is commercially available chitosan hemostatic powder, instant composite microporous polysaccharide hemostatic powder purchased from Success Bio-Tech Co., Ltd., with chitosan as its main active ingredient.

Ear marginal artery hemostasis model: The hair on the dorsal side of the rabbit ear was shaved off. The ear artery was transected at a position 7 cm away from the ear tip, and blood immediately gushed out from the wound. After free bleeding for 5 s, the superficial blood at the rabbit ear wound was gently wiped off with a sterile cotton ball. Subsequently, 150 mg of β-SFN-EA was immediately applied on the bleeding site, a pre-weighed cotton ball was vertically placed gently on top, timing was started right away, and the cotton ball was gently lifted every 30 s to check for persistent bleeding. If bleeding persisted from the wound, the cotton ball was placed vertically again and the wound was re-examined every 30 s until hemorrhage ceased. The bleeding time was recorded, and the effused blood was absorbed with cotton balls. The control group was treated with 150 mg of commercially available chitosan hemostatic powder, while the blank control group was treated only with cotton balls. All other steps remained consistent with the above description. After hemostasis was achieved, the cotton balls were weighed, and blood loss was calculated by the gravimetric difference method. Surviving rabbits were euthanized. Each sample was tested repeatedly for six times, and a mean value was adopted.

Liver hemostasis model: After the rabbit was anesthetized, the abdominal hair was shaved off. The rabbit was placed in a supine position. The abdomen was disinfected with 75% medical alcohol, followed by laparotomy. The left medial lobe of the liver was exteriorized from the abdominal cavity and laid on sterile gauze. A wound measuring 3.0 cm in length and 0.5 cm in depth was created on the liver. After free bleeding for 5 s, 50 mg of β-SFN-EA was immediately spread over the bleeding site. A pre-weighed cotton ball was vertically placed gently on top, timing was started right away, and the cotton ball was gently lifted every 30 s to check for persistent bleeding. If bleeding persisted from the wound, the foregoing procedures were continued until bleeding stopped. The bleeding time was recorded, and the effused blood was absorbed with cotton balls. The control group was treated with 50 mg of commercially available chitosan hemostatic powder, while the blank control group was treated only with cotton balls. After hemostasis was achieved, the cotton balls were weighed, and blood loss was calculated by the gravimetric difference method. Surviving rabbits were euthanized. Each sample was tested repeatedly for six times, and a mean value was adopted.

Femoral artery hemostasis model: After anesthesia, the rabbit was placed in a supine position, and the hair on the hind legs was shaved. The hind legs were disinfected with 75% medical alcohol, followed by blunt dissection of the skin and soft tissues using a surgical scalpel to expose the femoral artery. The femoral artery was completely transected. After free bleeding for 5 s, the superficial blood at the wound was gently wiped off with a sterile cotton ball. Subsequently, 150 mg of β-SFN-EA immediately spread over the bleeding site, a pre-weighed cotton ball was vertically placed gently on top, timing was started right away, and the cotton ball was gently lifted every 30 s to check for persistent bleeding. If bleeding persisted from the wound, the cotton ball was placed vertically again and the wound was re-examined every 30 s until hemorrhage ceased. The bleeding time was recorded, and the effused blood was absorbed with cotton balls. The control group was treated with 150 mg of commercially available chitosan hemostatic powder, while the blank control group was treated only with cotton balls. After hemostasis was achieved, the cotton balls were weighed, and blood loss was calculated by the gravimetric difference method. Surviving rabbits were euthanized. Each sample was tested repeatedly for six times, and a mean value was adopted.

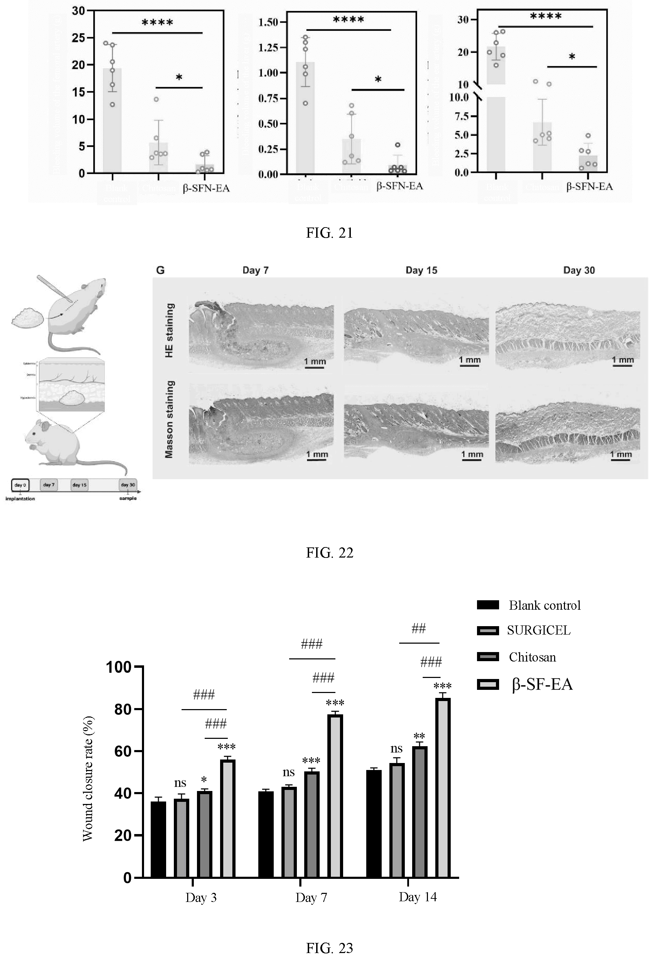

FIG. 20 and FIG. 21 are the comparison charts of bleeding time and bleeding volume for rabbits.

An artery runs along the dorsal side of the rabbit ear, which is comparable in diameter to the aorta in an adult human arm. Conventional hemostatic materials can hardly achieve effective hemostasis here, making the rabbit ear an ideal model for evaluating the hemostatic performance of biomaterials. In the ear marginal artery experiment, both the bleeding time and the bleeding volume were significantly lower in the β-SFN-EA group than those in the chitosan group (*P*<0.05). In terms of bleeding time, the average hemostasis time in the chitosan group was 209.0 s, while that in the β-SFN-EA group was 111.5 s, a reduction of 87.44%; in terms of bleeding volume, the average bleeding volume in the chitosan group was 6.69 g, while that in the β-SFN-EA group was 2.24 g, a reduction of 198.66%. It can be seen that β-SFN-EA has a more obvious hemostatic effect.

Blood vessels in the human liver are the most abundant among all visceral organs, and hepatic hemorrhage is also a highly intractable problem during surgical procedures. Therefore, the rabbit liver hemorrhage model is representative for simulating visceral hemorrhage in humans. We created a wound 3.0 cm in length and 0.5 cm in depth on the rabbit liver to induce massive hemorrhage. β-SFN-EA exhibited remarkable absorbency against spurting blood, whereas the commercially available chitosan hemostatic powder failed to achieve comparable performance, showing a statistically significant difference (P<0.05). Interestingly, β-SFN-EA not only adhered to the wound to prevent secondary hemorrhage, but also could act as a protective barrier to isolate the injured organ from surrounding tissues and thereby avoid postoperative adhesions. In terms of bleeding time, the average hemostasis time in the chitosan group was 123.2 s, while that in the β-SFN-EA group was 76.0 s, a reduction of 61.84%. In terms of bleeding volume, the average bleeding volume in the chitosan group was 0.35 g, while that in the β-SFN-EA group was 0.07 g, a reduction of 400%.

Among hemorrhages occurring in human extremities, femoral artery hemorrhage in the thighs features the fastest blood loss and carries the highest risk factor. The femoral arteries at the proximal thighs of rabbits have a diameter comparable to that of the lower leg arteries in adult humans. Accordingly, the rabbit femoral artery hemorrhage model is representative for simulating severe massive hemorrhage from human extremities. We established a lethal femoral artery injury model in rabbits during the surgery. The control group failed to achieve a macroscopically visible hemostatic effect, and could achieve complete hemostasis only with the help of compression. In contrast, the β-SFN-EA group realized spontaneous bleeding cessation without any external force applied, exhibiting significantly superior performance compared with the chitosan group (P<0.05). In terms of bleeding time, the average hemostasis time in the chitosan group was 246.0 s, while that in the β-SFN-EA group was 92.0 s, a reduction of 167.39%. In terms of bleeding volume, the average bleeding volume in the chitosan group was 5.67 g, while that in the β-SFN-EA group was 1.64 g, a reduction of 245.73%. In summary, the massive hemorrhage models for rabbits verified that β-SFN-EA had a remarkable effect in rapid hemostasis.

Meanwhile, it was seen from all of the three rabbit hemorrhage models that β-SFN-EA exhibited a higher settlement rate in blood compared with other materials and could rapidly settle to a bleeding site compared to other drugs, while the commercially available chitosan powder was lighter and more likely to float on the surface of the whole blood and be washed away by the blood flow, thereby affecting their hemostatic effect.

### Embodiment 21: Evaluation of in vivo biocompatibility

Subcutaneous implantation was performed on the dorsal region of SD rats for 7, 15 and 30 days, followed by histopathological examination to evaluate the in vivo biocompatibility (FIG. 22). H&E staining revealed that acute inflammation appeared at the particle-tissue interface after 7 days, inflammation and collagen deposition were alleviated after 15 days, and no residual materials or obvious tissue reactions were observed by day 30. These results indicated an initial inflammatory response followed by fibroblast proliferation and accelerated healing, and complete absorption of β-SFN-EA after 30 days.

### Embodiment 22: Evaluation of tissue healing effect

### (1) Experiment of full-thickness skin defects in mice

A full-thickness skin defect wound model was adopted to evaluate the effect of a single administration of β-SF-EA on subsequent cutaneous wound healing. C57BL mice were divided into four groups: blank control group, β-SFN-EA group, chitosan group and SURGICEL (Johnson & Johnson) group. Mice were anesthetized using an inhalation anesthesia machine, followed by dorsal hair removal and disinfection of the dorsal area with iodophor and alcohol. Subsequently, healing dressings were sutured onto the backs of the mice, and a circular skin defect wound (d=8mm) was created within the dressing area on the back of each mouse using a skin biopsy punch. On day 0, 10 mg of the drug was administered to each group, except the blank control group, which received no treatment. All the wounds were fixed with breathable medical dressings to prevent the drug from moving. On days 0, 3, 7 and 14, the wound was photographed using a digital camera and the dressing was changed. The size of the wound was measured using the Image J software. Wound healing rate (%)=((Aₒ-Aₜ)/Aₒ)*100%, where Aₒ is the initial wound area, and Aₜ is the wound area at different time points (days 3, 7, 14). To assess the healing process, four mice were randomly selected from each group and euthanized on days 3, 7 and 14, and the skin and muscle tissues at the wound sites were harvested and fixed in 4% paraformaldehyde for 24 h. Subsequently, the harvested skin was embedded in paraffin and stained with H&E and Masson. The healing status was evaluated by analyzing indicators including epithelial thickness, epithelial integrity, scab formation, neovascularization, inflammatory cell infiltration, and collagen fiber deposition. The Image J software was used to quantify the density of collagen deposition. Using the immunofluorescence detection of interleukin-6 (IL-6) and vascular endothelial growth factor (VEGF), we investigated the anti-inflammatory and angiogenic effects of β-SFN-EA on the wound area. Immunohistochemical staining was performed to detect the expression levels of IL-6, tumor necrosis factor-α (TNF-α) and platelet endothelial cell adhesion molecule-1 (CD31) in skin wounds. The Image J software was used to analyze positive markers.

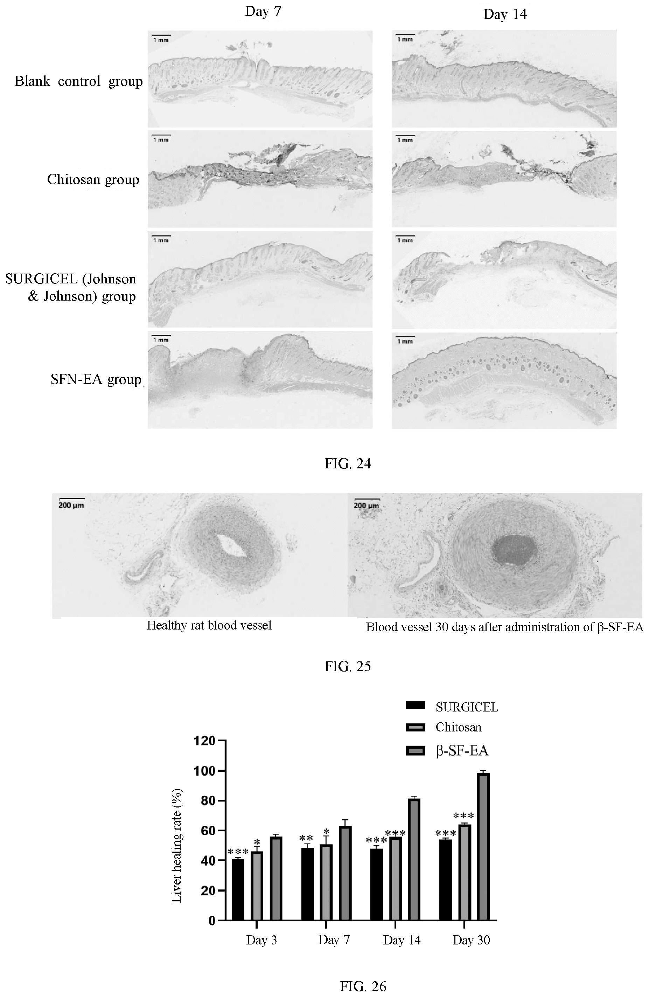

As shown in FIG. 23, the calculated wound closure rates on days 0, 3, 7 and 14 visually reflect the therapeutic efficacy. After 3 days of treatment, the wound area in all groups decreased to varying degrees. Compared with the β-SF-EA group, the blank control group exhibited the lowest wound closure rate, indicating the poorest healing effect. Compared with the chitosan group and the SURGICEL (Johnson & Johnson) group, the β-SF-EA group achieved the highest wound closure rate. On day 7, the β-SF-EA group still maintained the highest wound closure rate among all groups, demonstrating the optimal therapeutic efficacy. The H&E-stained sections of wounds harvested on day 7 of healing are presented in FIG. 24. Compared with the blank control group, the chitosan group exhibited abundant fibroblast proliferation, yet the defective tissue was not fully filled. Sparse newly formed capillaries were scattered throughout the granulation tissue, demonstrating a moderate repair capacity. Meanwhile, massive inflammatory cells infiltrated around native blood vessels, and the overall tissue morphology of the sections remained irregular; compared with the blank control group, the SURGICEL (Johnson & Johnson) group exhibited obvious tissue damage accompanied by extensive inflammatory cell infiltration in sections. In addition, massive fibroblasts and granulation tissue formation were not observed, showing a negligible repair capacity; compared with the blank control group, the β-SF-EA group not only produced a large number of fibroblasts and granulation tissues, but also had a dense network of new capillaries, which was conducive to the repair of the wound surface. Among the three groups, the SURGICEL (Johnson & Johnson) group displayed the weakest repair effect, and the chitosan group showed a slightly improved repair capacity yet suffered from severe inflammatory cell infiltration and incomplete tissue reconstruction, while the β-SF-EA group achieved optimal tissue repair with mild inflammatory response, abundant fibroblasts and granulation tissues, all of which facilitated wound regeneration. On day 15, fibroblasts and granulation tissues were further increased in the chitosan group, accompanied by more newly formed capillaries that accelerated the wound repair process, but the overall tissue structure of the sections remained irregular, and inflammation was alleviated compared with day 7. Obvious tissue defects could still be observed in the SURGICEL (Johnson & Johnson) group, indicating that SURGICEL (Johnson & Johnson) exerted a negligible repair effect. In the β-SF-EA group, the number of fibroblast clusters and granulation tissues decreased relative to day 7, native tissues were partially restored, and inflammatory response was further attenuated.

### (2) Experiment on vascular tissue healing of rabbit femoral artery

New Zealand white rabbits were weighed and anesthetized, then fixed in a supine position. The hair on hind legs was shaved off, followed by disinfection of hind legs with 75% medical alcohol. A scalpel was used to dissect the skin and soft tissues to expose the femoral artery. A non-penetrating arterial notch was created on the femoral artery using a medical blood collection needle (0.7*25mm). Sterile medical cotton balls were used to absorb the gushing blood, followed by rapid administration of β-SF-EA. After hemostasis was observed, the material embedded in muscle tissue was removed, and the muscle and skin layers were sutured sequentially; for the blank control group, blood was not absorbed with cotton balls, the rabbits were placed in rabbit cages to lose blood naturally until they regained consciousness, and after death, residual blood was cleaned, body weight was measured, blood loss volume was calculated, and vascular specimens were collected for H&E staining to observe arterial tissue defects. Thirty days after the surgery, the rabbits were euthanized, and femoral artery tissues were harvested for H&E staining to evaluate the recovery of arterial layers.

The results in FIG. 25 show that the blood vessels in the β-SF-EA group were almost completely repaired on day 30; although mild vasoconstriction and luminal narrowing were observed, blood flow remained unobstructed, indicating intact blood supply; in terms of animal performance, the hind limbs maintained normal motor function. It can be concluded that β-SF-EA not only achieved hemostasis in non-penetrating femoral artery wounds of rabbits, but also exerted a remarkable effect on promoting vascular tissue repair.

### (3) Experiment on liver tissue healing

SD rats were anesthetized via intraperitoneal injection of 1% sodium pentobarbital. The anesthetized rats were fixed in a supine position on an anatomical table. A longitudinal incision was made along the ventral midline to open the abdominal cavity and expose the right hepatic lobe of each rat. The peritoneal fluid was blotted dry with clean gauze. A parallel wound approximately 2.0 cm in length and 0.5 cm in depth was created on the right hepatic lobe using a surgical blade. Immediately after bleeding from the wound, pre-weighed cotton was used to absorb the blood, followed by topical administration of 20 mg of samples (β-SF-EA, chitosan, and SURGICEL (Johnson & Johnson) respectively) onto the wound surface. The wound was compressed with cotton for 30 s, followed by observation for bleeding. If oozing persisted, the wound was compressed with cotton for another 30 s and re-examined. This procedure was repeated until hemostasis was achieved. Afterwards, the abdominal cavity of the rat was sutured, and an Elizabethan collar was fitted on each rat to prevent wound biting. All rats were housed individually. To assess the healing process, four rats were randomly selected from each group and euthanized on days 3, 7 and 14. Liver tissues at the wound sites were harvested and fixed in 4% paraformaldehyde for 24 h. Subsequently, the harvested skin was embedded in paraffin and stained with H&E and Masson to assess wound healing status, with special attention paid to hepatic fibrotic alterations.

As shown in FIG. 26, the liver wound closure rates on days 0, 3, 7 and 14 intuitively reflected the healing effect of each group. After 3 days of treatment, the wound area was reduced to varying degrees in all groups. Compared with the chitosan group and the SURGICEL (Johnson & Johnson) group, the β-SF-EA group exhibited the highest wound closure rate and optimal healing effect. On days 7 and 14, the β-SF-EA group still maintained the highest wound closure rate among all groups, demonstrating a superior therapeutic efficacy. Complete tissue repair was nearly achieved by day 30.

### Embodiment 23: Evaluation of in vivo anti-adhesion performance

The in vivo anti-adhesion effect of β-SF-EA was evaluated using a rat abdominal sidewall defect-cecum abrasion model. SD rats were anesthetized with sodium pentobarbital at a dose of 50 mg/kg, and the abdominal hair was completely shaved. Subsequently, a 5cm long incision was made along the midline of the abdominal wall using surgical scissors. The cecum was isolated, and its serosal surface was gently abraded with sterile surgical gauze until punctate hemorrhage appeared. A 1cm×2cm peritoneal defect was created on the corresponding lateral abdominal wall with a surgical scalpel. For the β-SF-EA group, 1 mL of β-SF-EA sample was applied onto the injured abdominal wall and cecum. For the negative control group, 1 mL of sterile normal saline was sprayed over the wound surface. For the positive control group, a commercially available polylactic acid anti-adhesion film was used to cover the peritoneal defect of the abdominal wall. Four rats in each group were euthanized on days 7 and 14. The peritoneum was opened to examine intra-abdominal adhesions. The adhesions formed between the cecum and abdominal wall were scored according to a standard scoring system (Table 7).

Severe and inseparable adhesions were observed in the negative control group on days 7 and 14. Reduced adhesion between the abdominal wall and cecum was detected in the positive control group, probably because the commercially available polylactic acid anti-adhesion film was applied as a solid sheet and failed to fully cover the traumatized tissue surface, which constitutes a major drawback of such conventional barrier materials for anti-adhesion treatment. No signs of adhesion were observed in the β-SF-EA group on days 7 and 14. Further, the traumatized abdominal wall and cecum recovered to normal morphology in the β-SF-EA group on day 14. As shown in Table 7, the β-SF-EA group exhibited the lowest scores among the three groups, indicating that β-SF-EA had a favorable anti-adhesion effect.

**Table 7 Scores of Each Group on the Standard Adhesion Severity Scoring Scale**

| Adhesion score | Control | | β-SF-EA | | Film | |
|---|---|---|---|---|---|---|
| | 7^{th} | 14^{th} | 7^{th} | 14^{th} | 7^{th} | 14^{th} |
| 0 | 0 | 1 | 4 | 4 | 3 | 2 |
| 1 | 1 | 0 | 2 | 1 | 0 | 2 |
| 2 | 0 | 2 | 0 | 1 | 3 | 0 |
| 3 | 1 | 1 | 0 | 1 | 0 | 1 |
| 4 | 1 | 0 | 1 | 0 | 0 | 2 |
| 5 | 4 | 3 | 0 | 0 | 1 | 0 |
| Median | 5 | 3 | 0** | 0* | 2* | 1 |

| | | | | | | |
|---|---|---|---|---|---|---|
| Note: Control represents the blank control group, and Film represents a commercially available polylactic acid anti-adhesion film. Compared with the blank control group, **P*< 0.05, ***P*<0.05 | | | | | | |

## Claims

1. A silk fibroin complex loaded with a polyhydroxy phenolic compound, wherein the complex is prepared by self-assembly of the polyhydroxy phenolic compound and the silk fibroin; the silk fibroin is subjected to a β-sheet conformation transformation process, and the mass ratio of the polyhydroxy phenolic compound to the silk fibroin is less than 1:1.

2. The silk fibroin complex according to claim 1, wherein the mass ratio of the polyhydroxy phenolic compound to the silk fibroin is 1:2 to 1:16.

3. The silk fibroin complex according to claim 2, wherein the mass ratio of the polyhydroxy phenolic compound to the silk fibroin is 1:4.

4. The silk fibroin complex according to any of claims 1-3, wherein the polyhydroxy phenolic compound is one or more of resveratrol (RES), quercetin (QUE), baicalein (BAE), kaempferol (KAE), lignan, curcumin (CUR), ellagic acid (EA), chlorogenic acid (CGA), ferulic acid (FA), caffeic acid (CFA), syringic acid (SA), sinapic acid, rutin (RUT), myricetin (MYR), magnolol acid (MA), fisetin (FIS), puerarin (PUE), rhein (RHE), lonicerin (Lon), proanthocyanidins (PC), p-coumaric acid (p-CA), vanillic acid (VA), hesperetin (Hes), naringenin (NAR), luteolin (Lut), genistein (Gen), emodin (Emo), oleanolic acid (Ola), epigallocatechin gallate (EGCG), acaciin, tea polyphenols (TP), epigallocatechin (EGC), danshensu (DS), gallic acid (GA), pyrogallic acid (PA), catechin (C) and tannic acid (TA).

5. The silk fibroin complex according to any of claims 1-4, wherein the polyhydroxy phenolic compound is an insoluble polyhydroxy phenolic compound.

6. The silk fibroin complex according to claim 5, wherein the insoluble polyhydroxy phenolic compound is one or more of resveratrol, quercetin, baicalein, kaempferol, lignan, curcumin, ellagic acid, chlorogenic acid, ferulic acid, caffeic acid, syringic acid, sinapic acid, rutin, myricetin, magnolol acid, fisetin, puerarin, rhein, lonicerin, proanthocyanidins, p-coumaric acid, vanillic acid, hesperetin, naringenin, luteolin, genistein, emodin, oleanolic acid and epigallocatechin gallate.

7. The silk fibroin complex according to claim 6, wherein the insoluble polyhydroxy phenolic compound is ellagic acid.

8. The silk fibroin complex according to claim 7, wherein the mass ratio of the ellagic acid to the silk fibroin is 1:2 to 1:16.

9. The silk fibroin complex according to claim 8, wherein the mass ratio of the ellagic acid to the silk fibroin is 1:4.

10. A preparation method for the silk fibroin complex in any of claims 1-9, comprising a step of silk fibroin conformation transformation and a step of self-assembly of the silk fibroin and the polyhydroxy phenolic compound: step (1): the silk fibroin is subjected to a conformation transformation process from α-helix to β-sheet to obtain a silk fibroin solution, wherein the conformation transformation can be achieved by either chemical or physical methods, the chemical methods include promoting the transformation through polyols, polylactic acid, metal ions, pH or hydroxypropyl methylcellulose, and the physical methods include promoting the transformation through high temperature, hydration and pressure, ultra-low temperature storage, lyophilizing, shear force, ultrasound, eddy current, laser irradiation, and high-pressure carbon dioxide treatment; step (2): after being dissolved or suspended, the polyhydroxy phenolic compound is mixed with the silk fibroin solution obtained in step (1) and stirred to induce self-assembly, thereby obtaining a silk fibroin complex loaded with the polyhydroxy phenolic compound, wherein the feeding ratio of the polyhydroxy phenolic compound to the silk fibroin is a mass ratio of less than 1:1.

11. The preparation method according to claim 10, wherein in step (2), the feeding ratio of the polyhydroxy phenolic compound to the silk fibroin is a mass ratio of 1:2 to 1:16.

12. The silk fibroin complex according to claim 11, wherein in step (2), the feeding ratio of the polyhydroxy phenolic compound to the silk fibroin is a mass ratio of 1:4.

13. The preparation method according to any of claims 10-12, wherein in the step (1), a polyol is used to promote the transformation of the β-sheet conformation of the silk fibroin.

14. The preparation method according to claim 3, wherein the polyol is ethanol.

15. The preparation method according to any of claims 10-14, wherein the polyhydroxy phenolic compound is an insoluble polyhydroxy phenolic compound, the insoluble polyhydroxy phenolic compound in step (2) is dissolved or suspended in an organic solvent, and the organic solvent is selected from methanol, ethanol, propanol, propylene glycol, glycerol, n-butanol and isobutanol.

16. The preparation method according to claim 15, wherein the insoluble polyhydroxy phenolic compound is ellagic acid, the organic solvent is propylene glycol, and after the ellagic acid suspension is incubated with the silk fibroin in step (2), the final concentration of propylene glycol in the resulting solution is 5% to 20%.

17. The preparation method according to claim 16, wherein the final concentration of propylene glycol is 10%.

18. A silk fibroin complex loaded with a polyhydroxy phenolic compound prepared by the preparation method in any of claims 10-17.

19. A use of the silk fibroin complex in any of claims 1-9 and 18 in the preparation of a formulation with a hemostatic efficacy, wherein the formulation comprises the silk fibroin complex in any of claims 1-9 and 18, and pharmaceutically acceptable excipients.

20. The use according to claim 19, wherein the dosage forms of the formulation with a hemostatic efficacy include gauze, sponge, spun fiber, spray, powder, granule, gel, sealant, ointment, film, patch and suppository.

21. A use of the silk fibroin complex in any of claims 1-9 and 18 in the preparation of a formulation with the efficacies of wound healing, and/or infection resistance, and/or adhesion resistance, wherein the formulation comprises the silk fibroin complex in any of claims 1-9 and 16, and pharmaceutically acceptable excipients.
